# EUROPEAN PATENT SPECIFICATION

(11) **EP 0 842 042 B1**
(45) Date of publication and mention of the grant of the patent: **23.06.2004**
(21) Application number: 96910820.8
(22) Date of filing: 12.04.1996
(51) Int. Cl.: B32B 5/16, B32B 15/02, B32B 17/02, H01F 1/44, G01N 33/543

(54) **IMPROVED METHODS FOR THE MANUFACTURE OF MAGNETICALLY RESPONSIVE PARTICLES**
VERBESSERTES VERFAHREN ZUR HERSTELLUNG MAGNETISCH EMPFINDLICHER TEILCHEN
PROCEDES AMELIORES DE FABRICATION DE PARTICULES A SENSIBILITE MAGNETIQUE

(30) Priority: 07.06.1995 US 482448
(43) Date of publication of application: 20.05.1998
(73) Proprietor: IMMUNIVEST CORPORATION, Wilmington, DE 19899 (US)
(72) Inventor: LIBERTI, Paul, A., Huntingdon Valley, PA 19006 (US); RAO, Galla, C., Princeton, NJ 08540 (US); CHIARAPPA, Joseph, N., Flemington, NJ 08822 (US); PICCOLI, Steven, P., Salberry, Connecticut 06488 (US)
(74) Representative: Kiddle, Simon John
(86) International application number: PCT/US1996/005058
(87) International publication number: WO 1996/040502

(56) References cited:
- WO-A-91/02811
- DE-A- 4 142 405
- US-A- 4 795 698

## Description

### FIELD OF THE INVENTION

This invention pertains to stable suspensions of magnetic particles and to resuspendable coated magnetic particles, preferably, but not limited to, those having biochemical or biological activity, to compositions including such particles, and to methods of making and using such particles and compositions.

Biologically active magnetic particles find use in a variety of preparative and diagnostic techniques. Among these is high gradient magnetic separation (HGMS) which uses a magnetic field to separate magnetic particles from suspension. In instances where these particles are attached to biological materials of interest (e.g., cells, drugs), the material of interest or target material may thereby be separated from other materials not bound to the magnetic particles.

As used herein, the term "resuspendable coated particle" refers to a finely divided solid, which forms a colloidal suspension and may be separated from the suspension and subsequently resuspended. "Magnetic" encompasses material which may or may not be permanently magnetic, which also may be paramagnetic or superparamagnetic but which in all cases exhibits a response in a magnetic field, i.e., is magnetically responsive. "Disrupted" particles are those which are too small to contain a complete magnetic domain or, alternatively, whose Brownian energy exceeds their magnetic moment. Generally, these particles are less than 0.03 micron in size.

### DESCRIPTION OF RELATED ART

Many techniques have been suggested in the prior art for the preparation of magnetic particles or organo-magnetic materials. Such particles generally fall into three categories: large, small and microagglomerates of small particles. Large magnetic particles, having diameters greater than 10 microns (µ), respond to weak magnetic fields and magnetic field gradients. Because of their size, they tend to settle rapidly from solution and also have limited surface area per unit weight. Large particles also tend to aggregate after they have been subjected to a magnetic field because they can be permanently magnetized. Small particles which have magnetic cores of mean diameter less than 0.03 µ remain in solution by virtue of their Brownian energy and hence do not spontaneously settle. Microagglomerates of such small magnetic particles have been prepared by various methods. Depending on the size of the microagglomerates, materials which can remain in solution for reasonable periods of time can be prepared. Additionally, the magnetic properties of small particles and microagglomerates of small magnetic particles are significantly different from those of the larger permanently magnetizable particles. Small magnetic particles composed of either single crystals of ferromagnetic materials such as iron oxides or agglomerates of such crystals become "super-paramagnetic" when the crystal size of the ferromagnetic materials is below about 0.03 µ. Unlike ferromagnetic crystals, superparamagnetic crystals only exhibit magnetic behavior when they are in a magnetic field gradient and do not become permanently magnetized. Such materials have been referred to as dispersible magnetic metal oxide particles and also as magnetically responsive particles.

One possible route to obtaining a magnetic particle bearing a bioreceptor is that of U.S. Patent Nos. 3,970,518 and 4,018,886 to Giaever, which teach the physical coating of such materials onto the magnetic particles via adsorption. The coating of bovine serum albumin onto 1 µ diameter nickel particles is exemplified.

U.S. Patent No. 4,230,685 to Senyei et al. considers the teaching of U.S. Patent No. 3,970,518 and states that there is "no literature verification that uncoated magnetic particles can effectively be made to bind with antibody" and presumably other bioreceptors. U.S. Patent No. 4,554,088 to Whitehead et al. states that antibodies adsorbed on iron oxides are substantially detached by 24-hour 50° incubation in 1M sodium chloride and also that the quantity of adsorbed material is low.

With respect to one type of superparamagnetic particle described herein, namely, colloidal particles, the method of recovery proposed in U.S. Patent Nos. 3,970,518 and 4,018,886 to Giaever could not easily be made to work on such colloidal particles, as the field strength required to capture such particles for washing away unadsorbed materials would be enormous. Additionally, a field gradient is required, which is not achievable with the apparatus there described. In conjunction with the preparative use of high gradient magnetic separation (HGMS), the concept of Giaever might work where there are effective means for adsorbing and retaining antibodies or bioreceptors on such particles.

In view of the apparent inability to produce functionally acceptable magnetic particles via bioreceptor adsorption, a number of other approaches have been pursued. These include U.S. Patent No. 4,230,685 to Senyei et al., which discloses the preparation of microspheres containing magnetite, albumin, and protein A. The preparation taught by Senyei involves an emulsion polymerization of the above ingredients. U.S. Patent No. 4,554,088 to Whitehead et al. teaches the silanation of magnetic metal oxides which subsequently can be covalently linked to bioactive molecules. Both of the immediately preceding approaches deal with agglomerated superparamagnetic particles; hence, the agglomerated materials are classed as magnetically responsive. Other patents which may be considered to be of interest include U.S. Patent No. 4,152,210 to Robinson et al.; 4,335,094 to Mosbach; 4,070,246 to Kennedy et al.; and 4,454,234 to Czerlinski. While these patents disclose the preparation or use of magnetic-biologic particles, none of these are considered to be similar to those of the present invention.

U.S. Patent No. 4,452,773 to Molday discloses "colloidal" iron oxide particles coated with non-ionic polysaccharide by forming magnetite in 25% (w/w) polysaccharide solutions. Molday further teaches the covalent linking of bioactive molecules to such formed particles by well-known chemical linking technology. U.S. Patent No. 4,795,698 to Owen et al., which is incorporated by reference herein, teaches the preparation of colloidal sized metal oxide particles which are coated in what is believed to be essentially a covalent manner by polymers or proteins which have substantial numbers of unpaired electrons. Bioactive molecules such as antibodies or enzymes retain biological activity in the Owen et al. process which involves (1) the coprecipitation of transition element oxides and polymer or protein at 0.1 to 1 mg/ml by titration with base to slightly alkaline pH, (2) the subsequent washing of the coprecipitate and (3) the resuspension of the coprecipitate in appropriate buffers followed by mild sonication resulting in colloidal magnetically responsive particles. In that process, nearly all of the polymer or protein is precipitated. When the preparation of dextran-coated particles was attempted according to the process conditions of Owen et al., a resuspendable colloidal particle could not be obtained. That result, along with the fact that Owen et al. requires coprecipitation with polymers having substantial numbers of unpaired electrons (in contrast to dextran and the other nonreactive polysaccharides which Molday teaches) which apparently interact directly with transition metals, indicates that the processes of Molday and Owen et al. are substantially different. It should also be noted that the Owen et al. process requires the protein or polymer to be in water or low ionic strength buffer.

In view of the apparent absence of interaction of ferric and ferrous ions with dextran and the nature of the Molday dextran particle, it is instructive to examine the process taught therein. The colloidal particles of Molday are prepared by forming magnetite from ferric and ferrous chlorides with NH₄OH in the presence of 25% (w/w) aqueous Dextran T-20 (Pharmacia) or other similarly concentrated polysaccharides. Aggregates formed in the process are subsequently removed by three cycles of centrifugation at low G force. Colloidal dextran magnetite in the supernatant is recovered following gel filtration which separates the unreacted dextran from the colloidal particle which appears in the void volume. A substantial quantity of free dextran exists after the particles are formed. Although there is no discussion of the mechanism for colloidal dextran formation, it seems reasonable to suggest that dextran plays a physical or "barrier" role in the process. The basis for this suggestion is that 25% (w/w) dextran solutions are extremely viscous with extensive hydrogen bonding due to the interaction of its abundant hydroxyl groups with water. These factors create a system which limits diffusion. This would enable ferric and ferrous ions interacting with base to form local crystal nucleation sites whose growth will be related to the ability of bystander ions to participate. The presence of dextran could thus limit ionic participation resulting in the formation of small magnetite crystals (less than 300Å) which then are capable of adsorbing dextran molecules onto their surfaces. Thus, in this scenario, the dextran plays several roles in the process.

An alternative mechanism for formation of the Molday dextran magnetite is related to a fundamental property of magnetite. From the electron micrographs of Whitehead et al. and from the descriptive material of Senyei et al., it appears that magnetite prepared by the usual base precipitation of iron chlorides is composed of stable crystal of about 300Å or less in size. In view of the absence in the literature of reports indicating that magnetite can be made into a stable colloidal dispersion, it would seem that these crystals have a strong tendency to aggregate via mutually attractive molecular forces. On the other hand, by forming crystals "in situ" and in "chambers" formed by the high concentration of dextran employed by Molday and by having the "walls" of these individual chambers being able to collapse onto and coat such crystals, Molday appears to have been able to achieve the production of a colloidally stable magnetic particle. In view of the immense difference in surface areas provided by such individual magnetite crystals in contrast to the materials of Giaever, Molday appears to have additionally serendipitously solved the adsorption problem for certain polysaccharides.

A colloidal dispersion of magnetic particles in rocket fuel is disclosed in U.S. Patent No. 3,215,572 to Papell. The dispersion is said to include magnetic particles, such as magnetite (Fe₃O₄), 0.25µ in diameter and smaller, preferably less than 0.10µ in diameter. The dispersion is produced by ball milling a suspension of larger particle size magnetic particles in the propellant, with a grinding agent, which prevents "agglomeration or welding of the minute particles as grinding progresses." (column 2, lines 33-34). The ball mill includes metal balls to produce the grinding action. The grinding agent, generally included at levels on the order of 2% but possibly 10%, typically comprises oleic acid; further "... other grinding agents such as stearic acid and cetyl alcohol may be utilized in the production of a magnetic propellant and other long chain hydrocarbons having similar high surface tensions, such as benzene, ethane, hydrazine and gasoline may be utilized as the particle carrier and major constituent of the magnetic propellant," (column 4, lines 5-6).

US application Serial No. 397,106 discloses a method for producing polymer/protein coated magnetic particles which involves disrupting pre-formed crystal agglomerates (magnetite related transition element oxides) in the presence of coating material to produce materials which are in the 25 nm to micron size range. The size of the resultant product depends on the degree and conditions for disruption and the ratio of coat material to crystal agglomerates. Sonication under various conditions is disclosed as the method of choice. For polymer coated materials this process has major advantage over those where metal oxides are formed *in situ* in the presence of coat material, such as in Molday or Owen. By separating the process of preparing transition element oxide crystals from the coating step interference of coat material in the former process is avoided. Such interference can result in a variety of disadvantages such as heterogeneous crystal growth, difficulty in controlling the oxide process and imperfections in crystals, all of which can compromise the final product.

U.S. application Serial No. 08/231,379, which is a CIP of US application Serial No. 397,106 relates to another modification which in many respects constitutes an improvement upon the above described process, wherein crystal agglomerate disruption is performed in the absence of coating material. That modification has advantage when material to be coated is adversely affected by the disruption process. Another advantage of separating the disruption from the coating step is that the presence of coating material can inhibit disruption by linking two crystal agglomerates together thus interfering with the disruption process.

Despite the simplicity of the processes described in the last mentioned patent applications and the utility of the resultant material, materials derived from these processes are limited in certain respects. One limitation of these materials is their loss of stability in moderate (0.01M) ionic strength buffers which causes them to agglomerate and eventually settle from solution. Thus in the manufacturing process variation in ionic strength which leads to agglomeration should be avoided. Further such materials will generally only resuspend after magnetic collection if they are in buffers of low ionic strength. Even then, repeated magnetic collection and resuspension can lead to agglomerates. This property can, however, be advantageous as in the case of doing immunoassays in serum or ionic strength buffers near 0.15M. Since these media lead to some agglomeration during the incubation period of the assay and since agglomerates require smaller magnetic gradients to be pulled from solution, this property which is undesirable in some instances becomes an actual benefit and permits the use of material of smaller size than would be the case in the absence this phenomenon or alternatively allows the use of lower magnetic gradient separation devices.

On the other hand, there are many applications where size integrity of the magnetic colloid is very important and where such agglomeration is highly undesirable. One example would be the use of these materials in laboratory or bioprocessing isolations of monoclonal antibodies (MAbs) from ascites fluid or from culture. Typically such media are at physiological ionic strength (0.15 M) and, for example, when Protein A is used to bind MAbs and subsequently desorb MAbs, buffers of such ionic strength or considerably higher are used. There are also instances in immunoassay where agglomeration is not desirable, such as dual stage incubations. For example, to assay for chronic hepatitis, a typical approach would involve incubating ferrofluid specific for human IgM with patient serum so as to capture the IgM in the sample. Capture would be accomplished by magnetically separating the capture material and subsequently washing out non specific proteins. Next the ferrofluid bearing patient IgM would be incubated with excess labeled hepatitis antigen, separated again, washed and the label detected by some appropriate means. The dual incubations of this process and the two separations require a material that will be stable to both moderate ionic strength and to multiple magnetic separations and resuspensions.

During the course of extensive research and development on the above processes, several observations have led to the hypothesis that the problem of high ionic strength colloidal instability might be due to incomplete crystal agglomerate coverage by the coat polymer/proteins employed. Further, even though it is very difficult to calculate accurately the surface area of such agglomerates and the amount of coat material which should be adsorbed thereon as a monolayer, calculation does suggest that incomplete coverage is a possibility. From extensive experience with dispersed magnetite crystal agglomerates, it is apparent that surface charge is critical to keeping the agglomerates dispersed. This can be seen by performing a simple experiment such as sonicating magnetite in low ionic strength phosphate buffer (10 - 20 mM). Such a process will result in a transitionally stable dispersion. If such material, while in the dispersed state, is magnetically collected it will be found to not resuspend. The dispersion can also quickly be agglomerated by merely increasing the ionic strength with simple salts. Further evidence suggesting that this process does not result in complete coating is: (1) anionic polymer, dextran or bovine serum albumin (BSA) coated materials non-specifically stick to mammalian cells, and binding can in part be diminished with anionic polymers which can compete with cell surface sialic acid for "bare spots" which would have positive charge due to iron atoms at the crystal surfaces (see US patent application no. 07/976,476); (2) a substantial portion of material collected by HGMS using very fine steel wool (gradients in excess of 150 kGauss/cm) aggregates in the process; and (3) the size of materials is affected by ionic strength and at ionic strengths as low as 0.02 M agglomeration can be observed.

It would be desirable to have a well coated particulate base material for several reasons. By coating material to a greater degree with a hydrophilic coating, stability in high ionic strength media should be achievable, covering "bare spots" should reduce non-specific binding to cell surfaces, and better coated material should generally permit greater amounts of bioreceptor to be coupled yielding higher bioactivity. There are also process advantages that should accrue since better coated materials can be magnetically collected and resuspended without crystal-crystal interaction which will lead to aggregation. The independence of such processes from concerns of high ionic strength induced aggregation also would be of significant benefit in various manufacturing steps such as purification. Furthermore, it is simpler to employ magnetics to remove unbound reagent in some particular step rather than to employ column chromatography.

### SUMMARY OF THE INVENTION

This invention relates in part to new methods for simplified production of magnetically responsive superparamagnetic particles. According to one aspect of the invention, a process is provided whereby magnetically responsive metal oxides can be coated effectively by employing in the coating process means for disrupting crystalline agglomerates such that coating can take place while the resultant crystals are in the disrupted state. A wide range of materials (including dextran, proteins, synthetic polypeptides, polymers, copolymers, detergents and combinations thereof) can be coated onto such crystals resulting in colloidal magnetically responsive particles. In addition to obtaining a colloidal product, it is possible in most instances, by limiting the amount of coating material, to obtain stable microagglomerates which retain adsorbed material in an extremely effective fashion and which can be removed from solution with simple laboratory magnets.

In an alternative embodiment, this invention relates to the production of magnetically responsive superparamagnetic particles which are colloidally stable in high ionic strength systems, e.g. 1.0 to 2.0 M NaCl, and which can repeatedly be subjected to high gradient magnetic separation and resuspension without growth in size as would be evidenced by the appearance of turbidity or particle size growth. Such materials provide process advantages in manufacture which significantly decrease cost of production. These advantages include the ability to repeatedly separate the resultant particles from reagents via magnetic separation rather than using column chromatography, a significantly greater latitude in choice of buffers (types and buffer strengths) which can be used in these processes, as well as in coupling chemistries or modification/derivatization reactions. These materials also have a significantly greater ability to be filter sterilized (for materials below 200 nm) as regards the amount of product which passes such filters. Further, they are compatible with a greater choice of filter material. These materials also demonstrate significantly lower nonspecific binding, particularly to mammalian cells.

This new class of material also has significant application advantages such as ability to undergo repeated separation and resuspension as is often required for multi-incubation assays. Due to their increased content of coating material, they have a greater ability to couple greater amounts of bioligand to them. In such applications where the presence of these materials quenches or absorbs developed signal, such as in chemiluminescent immunoassays or nucleic acid detection, the higher biological activity results in the ability to use less material, which results in greater signal output. This higher biological activity as well as colloidal stability under a wide range of conditions typically found in biological and bioprocessing systems and likely to be found in various other manufacturing applications or processes gives these materials significant advantage over magnetic-polymer particles made by previously available procedures. These materials indeed represent a significant advance over the prior art for coating clusters of crystals of various transition element oxides, and particularly magnetite.

The magnetic particles of the invention are prepared by a direct coating process performed on a transiently stable particulate magnetic substrate, which is further described hereinbelow. In carrying out this process , a particulate magnetic starting material is divided into a plurality of smaller sized particles with the ability to aggregate, thereby providing a bare or uncoated particulate magnetic substrate. The particulate magnetic substrate thus obtained, which is suspended in a suitable liquid medium, is then contacted with an appropriate coating material to form a mixture, before substantial particulate magnetic substrate aggregation occurs, for a time sufficient for the coating material to adhere to the substrate particles, thereby yielding the desired resuspendable, coated magnetic particles. In an alternative embodiment, the mixture may also be heated for a sufficient time to facilitate adherence of the substrate to the magnetic particles.

Thus, it is not essential to subdivide the particulate magnetic starting material in the presence of the coating material in order to obtain useful colloidal magnetic particles coated with a biologically active biofunctional ligand, for example. Rather, this process enables the coating of pre-formed, transiently stable particulate magnetic substrate. The period of stability of the substrate particles is readily determinable by routine experiment in the manner described below. This approach provides certain notable advantages, among which is avoidance of any deleterious effects that the selected disruption technique may have on the coating material. Moreover, sequential addition of coating materials eliminates certain operational restrictions inherent in the embodiment in which the particulate starting material is subdivided in the presence of the coating material. This may be of some importance where the primary coating material is present in a mixture with other substances having greater affinity for the magnetic particulate substrate. Also, because sequential coating affords greater control of the amount of coating material used, work up of the final product is greatly simplified.

The application of additional coating materials, e.g., a particle dispersion aid, to the particles bearing the primary coating material is also contemplated to be within the scope of this invention.

Also in accordance with the present invention, there is provided a method for selective binding of various polyelectrolytes, e.g., DNA or RNA, to the resuspendable, colloidal magnetic particles described herein, which may be used to advantage in performing an array of bioanalytical techniques, such as polymerase chain reaction (PCR) or nucleic acid sequencing. This method comprises providing chemically-treated, resuspendable, colloidal particulates, which have a surface charge that is opposite in polarity to charge on the polyelectrolyte, the surface charge on the chemically-treated, resuspendable, colloidal particulates being the same as or different from any existing charge on the colloidal particulates prior to chemical treatment, and contacting a sample comprising the polyelectrolyte with the chemically-treated, resuspendable colloidal particulates, whereby the colloidal particulates directly bind to the polyelectrolyte, thereby forming an agglomerated mass consisting essentially of the polyelectrolyte and the particulates.

Once the polyelectrolyte is captured on the chemically-treated, resuspendable magnetic particles, it is readily removed from the test sample by magnetic separation, using, for example, the apparatus and methods of U.S. Patent 5,186,827 or U.S. Patent 5,200,084, which are commonly assigned with the present application. The entire disclosures of both of the last-mentioned patents are incorporated by reference in the present specification as if set forth herein in full. Also substantially pure polyelectrolyte can be recovered by appropriate manipulation of ionic strength or pH. A suitable recovery procedure is exemplified below. In this way, extraction of nucleic acids from complex mixtures, such as cell lysates, can be facilitated.

The chemically-treated, resuspendable colloidal magnetic particles used in performing the above-described method for selective binding of polyelectrolytes are considered to be unique in that target recognition and separation capabilities are combined in the individual treated particles. That is to say, affinity between the polyelectrolyte and the chemically-treated surface of the colloidal magnetic particles is essentially due to physical interaction. No additional affinity reagents or procedures for their application to the magnetic particles is required. Further, the small size of the magnetic particles provides increased reaction kinetics, and allows many particles to be bound to the target molecule, enhancing the magnetic attractability of the target molecule.

Preferably, the means for disrupting the parent magnetic particle starting material in the suspension is sonication, but other mechanical or chemical means may also be used. These other "means" include, for example, heating, other forms of particle energization, such as irradiation, and chemical means, such as pH modification or combinations of these treatments. In particular, a combination of pH modification and sonication may be used.

According to another aspect of this invention, there is provided acid-treated, resuspendable, colloidal particles, having a maximum particle size below 0.2 microns, which bind directly to negatively charged polyelectrolytes, forming an agglomerated mass consisting essentially of the negatively charged polyelectrolyte and the acid-treated colloidal particulates.

According to yet another aspect of the present invention, there is provided a base-treated, resuspendable, colloidal particulates, having a maximum particle size below 0.2 microns, which bind directly to positively charged polyelectrolytes, forming an agglomerated mass consisting essentially of the positively charged polyelectrolyte and the base-treated colloidal particulates.

The methodology disclosed herein incorporates the surprising discovery that coating of polymer or protein on to such crystals is markedly affected and enhanced by heat. More specifically if the coating reaction is performed at temperatures well above those temperatures at which processes involving proteins normally are done, not only is significantly more coating achieved but a product which is colloidally stable in high ionic strength is obtained. Contrary to the notion that protein coating reactions are best done in the cold or at no higher than 37°C, it has been discovered that if magnetite slurries are mixed with protein such as BSA and heated to temperatures higher than 60°C, typically 7.5 to 80°C, and sonicated, a product results which is salt stable and which can be separated and resuspended repeatedly. It has further been discovered that if the sonication of such mixtures is done in the cold (0 to 5°C) such as described in U.S. application Serial No. 397,106, and the mixture subsequently heated to 75°C while still in the presence of excess coating material, the product obtained will have the same properties as described above. This result demonstrates that the conversion to a salt stable material is independent of the sonication treatment and clearly only depends on the high temperature coating reaction. To examine if the coating reaction can be done in two steps as disclosed in US application no. 08/231,379 where slurries of magnetite are first sonicated to a dispersion of crystal agglomerates - single crystals up to agglomerates as large as 200 nm, depending on the degree of disruption - and subsequently coated in an independent step, magnetite was dispersed by sonication at 5°C or at 75°C and subsequently coated with protein/polymer at 75°C. In both cases product results which is stable to high concentrations of simple salts, 2 M NaCl and to repeated magnetic separation and redispersion.

### DETAILED DESCRIPTION OF THE INVENTION

It is hypothesized that magnetic materials, or more generally, transition element oxides, in particle form, tend to have significant surface polarity, which is minimized by agglomeration of crystals of such materials. When these crystal agglomerates are subdivided or disrupted, they tend to become unstable, again forming crystal agglomerates over time. In accordance with the present invention, the nascent (and probably charged) surfaces of these sub-particles are stabilized by the coating material which is deposited on these surfaces after the parent particles are sub-divided, but before crystal agglomerates begin to form. For that purpose, the coating material may be chosen on the basis of its tendency to respond to the surface polarity of the deagglomerated magnetic particle and various coating materials will thus react differently with different particulate magnetic materials. If the treating or disrupting technique is, or includes, pH modification, the effect of pH modification on sub-particle surface polarity and coating material polarity may also be a consideration. The coating material is selected in each case with regard to its ability to adhere to, or be adsorbed on or otherwise modify a property of the surface of the deagglomerated or sub-divided particle so that the stability of the particle product of reduced size is retained, to provide a stable suspension thereof.

This invention provides significant advantages over Molday and Owen et al. for preparing colloidal materials, beyond the inherent simplicity of the method. For example, if it is desired to attach compounds to metal oxide particles where the compound has reactive groups or activated groups for subsequently attaching other materials, such as antibodies or enzymes, the Molday and Owen et al. procedures are limited insofar as the choice of such compounds is concerned. This is because these processes necessarily start by mixing such compounds with metal chlorides which themselves can react with the compounds and which also create acidic pH. Further, base addition, usually ammonium hydroxide, is required to form the metal oxides and can clearly have deleterious effects on a variety of activated chemical groups used for the subsequent coupling. By contrast,

Certain types of coatings, such as the long chain hydrocarbons suggested in the Papell patent, have a detergent effect. Such coatings can serve to stabilize the subdivided deagglomerated magnetic particles and produce a stable suspension, such as that taught in Papell. In accordance with the present invention, these suspensions are produced, from magnetite and with sonication as the disrupting means, several orders of magnitude faster than in the ball milling process described by Papell.

To produce resuspendable products, it is important to select a coating material which not only stabilizes the sub-divided magnetic particles, but does so with a coating which remains intact when the coated particles are removed from suspension, which is not the case with Papell.

In investigating means for coating disrupted particles it has been discovered that such reactions are markedly enhanced by heating to relatively high temperatures. Temperatures from about 45 - 50 °C are effective and temperatures as high as 85°C have been employed with 75°C appearing to be optimal. In the case of protein coatings and certain polymers which have secondary and tertiary structure, it is surprising that these materials can be treated in this manner. The resultant resuspendable products have the characteristics of showing low nonspecific binding to cells as well as macromoleules, have significantly higher levels of coat material than non-heat treated product and most of all have the unusual property that they maintain colloidal stability in high ionic strength buffers. Depending on the degree of heating (temperature or time,) materials which are stable in 0.5 M NaCl up to 2.0 M NaCl can be produced.

Magnetic compounds which may be used as the starting material in the present invention include the transition metal oxides, sulfides, silicides and carbides, optionally having different transition metals in a single magnetic compound, such as Gd₃Fe₅O₁₂. Preferred is the class of magnetic oxides known as ferrites, generally represented as MO•Fe₂O₃ in which M is Zn, Gd, V, Fe, In, Cu, Co, Mg, and in particular magnetite (FeO•Fe₂O₃).

In addition to the transition element-containing compounds described by Owen et al. and the ferrites noted above, a class of magnetic metal oxide which does not contain iron can be coated as described in this invention. These compounds include oxides of combinations of two or more of the following metal ions: Al(+3), Ti(+4), V(+3), Mn(+2), Co(+2), Ni(+2), Mo(+5), Pd(+3), Ag(+1), Cd(+2), Gd(+3), Tb(+3), Dy(+3), Er(+3), Tm(+3) and Hg(+1). They differ from ferrites in both appearance and magnetic susceptibility. The non-ferrites can take any color from white or yellow to green and even brown. This makes them particularly useful in spectrophotometric applications. Non-ferrites are generally less strongly magnetic than ferrites and; as such, pass through HGMS filters in magnetic fields capable of collecting ferrite based materials which permits selective magnetic retrieval.

The non-ferrous oxides can be employed in place of the metal oxides described by Whitehead et al. to produce silane coated magnetic particles which have the desirable properties given above. Similarly, when the chlorides (or sulfates) of such combinations are employed according to the methods taught by Molday or by Owen et al., coated product having very desirable magnetic and spectral properties can be obtained.

Coating materials which may be used are in aqueous suspension or solution. The coating material is usually a synthetic or natural polymer and may be a protein, a peptide or a nucleic acid. In principle, however, the coating material can be any substance which has affinity for the surfaces of such crystals and which is not adversely affected by exposure to the high temperatures.

To make stable suspensions of coated sub-particles of the magnetic starting material, the mixture may be treated in a number of ways to disrupt or sub-divide the magnetic particulate starting material. These include mechanical and chemical means, such as mild heat, vibration, irradiation, sonication, pH modification, or a combination of these. Of these, sonication is particularly preferred. During or following this process the system may also be heated, preferably to 75°C and maintained at that temperature until coating is maximized.

In investigating means for disrupting or sub-dividing the magnetic particulate starting material, it has been discovered that, even in the absence of coating material, starting material can indeed by divided into smaller size particles, albeit generally in a transitory condition insofar as stability is concerned. From this observation, it has been established that if coating material is added to a suspension of the smaller size particulate magnetic substrate in a suitable liquid medium, following division of the magnetic starting material and before substantial particulate magnetic substrate aggregation occurs, i.e., while the smaller size magnetic particles remain stably suspended, resuspendable coated magnetic particles can be produced. By "substantial particulate magnetic substrate aggregation" is meant that a substantial number of particles are no longer in their most finely divided state, as evidenced, in some cases, by a change in the appearance of the suspension from shiny to dull, which is indicative of particle aggregate formation. Alternatively, the occurrence of substantial particle aggregation can be determined by means of quasi-electric light scattering, when the average particle size is shown to increase significantly from its minimum size.

Coating materials can also be added sequentially after the disruption process such that material of particular interest is first added to the sub-divided material in a limited amount and allowed some time to adhere to the particulate substrate. The exact time will depend upon how long crystal reaggregation takes under the conditions of exposure to the specific coating material selected. Next, a different coating material whose essential functions are to coat portions of disrupted crystals left bare by the primary coating and to prevent agglomeration is added so as to stabilize the system. Various disperson aids may be used for this purpose. Suitable dispersion aids include, for example, conventional detergents, anionic, cationic and/or non-ionic surfactants, and the like. The selection of an appropriate dispersion aid will depend on the nature of the surface charge on the colloidal magnetic particles. The dispersion aid serves to help stabilize the colloidal, magnetic particles in suspension.

The sequential coating approach enables the uptake of coating material of interest to be maximized and in many cases substantially complete particle coating can be achieved. In this way, the amount of the primary coating material can be limited, allowing the coating process to be performed without the need for subsequent purification of unbound primary coating material from the desired coated magnetic particle product. Additionally, by carefully controlling the concentration and/or timing of subsequently added coating material, stable agglomerates of different size can be obtained. Another advantage of adding coating materials after disaggregation of the particulate magnetic starting material is that any adverse effect that the disruption process may have on the coating materials will be avoided. Further, the latitude of carrying out the coating operation in two distinct steps has a variety of obvious process advantages for particular situations.

We have also discovered that the employment of high gradient magnetic separation (HGMS) in the production of such particles gives a dimension to their production which heretofore has not been achieved or recognized. The Molday and Owen et al. processes utilize either centrifugation, gel filtration or "salting out" as manipulative procedures during the processing of colloidal materials. In the example herein, HGMS has been used to separate colloidal particles from unbound coating substance. In processes where it might be desirable to chemically couple substances to such particles, processing involving HGMS is both readily scaled and highly efficient.

HGMS can also be used in concert with tuned magnetic fields to fractionate preparations based on their magnetic susceptibility/particle volume ratios. This permits convenient fractionation and production of particle preparations of discrete sizes. When used as NMR contrast agents, the size of the particle plays an important role in the metabolic fate of the material. HGMS, again with tuned magnetic fields, can also be used to selectively capture particles where the magnetically responsive core contains transition element oxides having higher magnetic susceptibilities than others in the mixture. This concept could have utility in a system in which colloidal particles having different magnetic susceptibilities as well as different bioreceptors could be mixed with a sample and removed by sequential HGMS using increasing gradient field strength.

Not only can HGMS be used to separate unadsorbed coating or unreacted substances and byproducts from the colloid al, magnetic particle product, it can also be utilized for immobilizing coated magnetic products and performing reactions on the immobilized material. Thus, if it is desired to chemically modify a given coating, reactants can be added to magnetically immobilized material, the reaction performed in the magnetically immobilized state and excess reactant or other reaction products easily washed away. This concept, somewhat like peptide synthesis done on solid supports, lends itself nicely to sequential reactions.

Sub-division of the magnetic particulate starting material can also be performed in the absence of coating material, utilizing a subsequent heat driven coating step. This can be accomplished at temperatures ranging from 0°C up to 85°C and as above, various mechanical or chemical means can be employed. A preferred embodiment for the disruption of magnetic particulate material has been found to be disruption at 0 to 5°C in the presence of low concentrations of neutral phosphate buffer (5 to 30 mM) and employing sonication as the means for disruption. Keeping the temperature in this range seems to confer two advantages over higher temperature disruption as oxidation and subsequent magnetic compromise of the crystals is avoided at the lower temperatures and smaller crystal agglomerates can be obtained for the same energy input. By disrupting magnetic material in the absence of coating material, a distribution of crystal agglomerate sizes is produced, which are resuspendable. The mean of the distribution has been found to be related to energy input (time and power of sonication), to the presence of various chemical species before and/or during the disruption process, the magnetic saturation value of the material and the manner in which the crystals are prepared. For example, in the preparation of magnetite and other transition metal oxides, the rate of addition of base (or the nature of the base, e.g., NH₄OH vs NaOH) in forming the oxides from the chloride or sulfate salts results in crystals which vary in size and which can be disrupted to different degrees. For example, fast addition of NaOH to the sulfate salts of iron results in magnetite crystal clusters which typically are smaller than those formed either by slow addition or by employing NH₄OH to effect the oxidation. Once the distribution of crystal agglomerates is formed, coating material is rapidly mixed in at a concentration sufficient to arrest reagglomeration and the mixture is heated to drive the coating reaction. The coating material can be at the same temperature or at some higher temperature. According to a particularly preferred embodiment, the magnetic starting material is subdivided, with heating, in the absence of the coating material, and subsequently mixed with coating material at 75-80°C, with continued heating at 75°C for 30 to 40 minutes.

As those skilled in the art will realize there are several advantages in separating the disruption step from the procedure that prevents reagglomeration, followed by performance of the step which drives and completes the coating. Clearly if the disruption step is done at high temperature in the presence of coating material where the coating reaction clearly is more avid, then the presence of coat material should likely interfere with the disruption reaction. Even though high ionic strength colloidally stable material can be produced in this manner, the fact that so many and varied reactions are occurring at the higher temperature suggests that such a process would be difficult to control. On the other hand, performing the disruption reaction in the absence of coat material in principle should be a controllable process, given that one starts each preparative procedure with material which at the crystal level is very similar in size, structure and in the case of magnetic materials, of similar magnetic character. By arresting the disrupted system from reagglomeration by adding coating material in a sufficient quantity to prevent reagglomeration and then heating the system to drive the coat reaction, one minimizes the possibility that coat material will crosslink near neighbor crystal agglomerates.

Although the foregoing methods of the invention have been characterized as coating methods, such methods may aptly be considered extraction methods, depending on the particular application. Thus, the methods described herein may be beneficially utilized for the specific purpose of extraction of a target material from a complex mixture, such as isolation of environmentally hazardous materials from a waste stream, product recovery from a reaction mixture or the separation of a component of value from a mixture comprising generally worthless components.

Another application of the present invention which is in the nature of an extraction is the method of binding polyelectrolytes, e.g., nucleic acids, to colloidal magnetic particles. This method involves the use of the chemically-treated, resuspendable, colloidal magnetic particles for selectively binding to polyelectrolytes, e.g., DNA or RNA as a means for extracting such target molecules from a complex mixture. The ability of such resuspendable, colloidal magnetic materials to bind to polyelectrolytes, as well as the enormous surface area of such materials, coupled with their ability to be magnetically retrieved affords significant benefits when so used. This binding agent is conveniently prepared by subdividing the particulate magnetic starting material into a plurality of resuspendable, smaller sized particulates which are treated with a chemical agent that imparts the desired surface charge to such particulates. Disruption techniques such as sonication or pH adjustment may be used for this purpose, the former being preferred. Sonication not only produces disaggregation of the particulate magnetic starting material, but may also be used to cause simultaneous disruption of certain biological entities, such as cells or bacteria, whose contents includes the target molecule. Upon release via sonication, the target molecule becomes bound to the resulting colloidal magnetic particles, provided they have been subjected to appropriate chemical treatment.

In carrying out this process, the particulate magnetic starting material may be disrupted to form a transient colloid, as described herein, to which a test sample containing the target molecule to be bound is subsequently added, or the particulate magnetic starting material can be disrupted in the presence of the target molecule.

The examples set forth below demonstrate that treatment of subdivided magnetic particulates with various chemical agents, such as acid or base, for adjustment of pH (or ionic strength), markedly affects binding selectivity for various polyanions and polycations. Thus, it is clear that as a means for performing extraction, this procedure offers considerable specificity for the polyelectrolyte of interest. By adjusting solvent, buffer and salt conditions after the binding step and after the bound polyelectrolyte material has been magnetically separated, the target polyelectrolyte is readily recoverable in biologically active form. After recovery of the target polyelectrolyte, the particulate magnetic material may be recycled.

It should be appreciated that this process will function even with transition element oxide materials which have relatively low magnetic susceptibility properties as described hereinabove. In those cases where the material used for polyelectrolyte capture is not sufficiently magnetic for magnetic separation to be effective, filtration could be used for separation and subsequent recovery.

The following examples will serve to illustrate the principles of this invention; however, these examples should not be construed as limiting the scope of this invention.

All reagents and chemicals used in these experiments were of analytical grade and obtained from Fisher Scientific (Valley Forge, PA) unless otherwise specified.

### Comparative Example 1

Magnetite was prepared by mixing solutions of 3.0 and 1.5 mg/ml of ferric chloride hexahydrate and ferrous chloride tetrahydrate, respectively, with stirring at room temperature while raising the pH to 8.6 with NH₄OH. The resultant magnetite was collected magnetically, washed 3 times with distilled water and resuspended in distilled water. The preparation so made contained 1.5 mg/ml magnetite. Even after sonication (Fisher - Sonic Dismembrator Model 300) for 3 minutes at 70% output, these preparations will not remain suspended for more than about 2 minutes.

To coat magnetite particles by the method of this invention, 0.5 ml aliquots of various concentrations of various coating materials were mixed with 0.5 ml aliquots of the 1.5 mg/ml magnetite suspension. Samples were mixed in conical plastic centrifuge tubes and subsequently sonicated for 3 minutes at 70% output at room temperature. A positive or partially positive coating result was apparent from visual inspection of the manner in which light was scattered by the sample.

To determine the efficiency of coating, resultant samples were further observed for settling and also were fractionated into colloidal coated magnetite or microagglomerates thereof in the following fashion: 0.5 ml aliquots of the sonicated mixture in 12 x 75 mm test tubes were placed in a Ciba-Corning Magnetic Separator (Walpole, MA) and visually observed. The criterion used to determine if the coated magnetite crystals were colloidal was to observe if the resultant material remained in solution in the magnetic separator for a period of 10 minutes, i.e., in the magnetic supernatant. This criterion was established on the basis that solutions of protein coated colloidal magnetite prepared by the process of Owen et al. could not be separated magnetically in the Magnetic Separator when placed therein for such periods.

To determine what portion of the sonicated mixtures formed stable, coated, but agglomerated materials which pulled to the sides of the test tubes in the Magnetic Separator, materials so formed were washed with 20mM phosphate three times and resuspended in the same buffer. Their resuspension characteristics compared with uncoated magnetite were distinctly different, as they would remain suspended for hours compared with minutes for the latter.

A second criterion involved examining the magnetic supernatant as follows: Putative colloidal material was separated from the mother liquor by HGMS, washed, and resuspended in buffer, and visually observed for colloidal appearance and stability. HGMS was accomplished by placing approximately 20 mg of fine grade stainless steel wool (McMaster-Carr, New Brunswick, NJ) (washed in detergent, incubated in 1% BSA phosphate buffered saline (PBS), rinsed with deionized water, dried and cut in approximately 3 mm lengths) into 12 x 75 mm glass test tubes. 100 microliters of the supernatants to be tested were added to the test tubes containing the stainless steel wool and placed in the magnetic separating rack for 2 minutes by which time magnetic material had collected on the stainless steel wires. Clear non-magnetic supernatants were removed with Pasteur capillary pipettes and the magnetic material washed 3 times with 300 microliters of 20 mM phosphate (pH 7.5). After the third wash, collected magnetic material was resuspended in the phosphate buffer, after removing the tube from the magnetic rack, and examined visually for the appearance of a stable colloid.

In some instances recovered material was also sized by laser light scattering (Coulter Sub Micron Particle Analyzer N4SD, Hialeah, FL). Some supernatants were separated from unadsorbed material by gel filtration chromatography on Sephacryl-300 (Pharmacia) or ultra gel AcA-22.

Table I lists different compounds used in coating experiments, their concentration(s), and other solvent conditions. The presence and semi-quantitation of colloidal and agglomerated materials were determined visually by the procedures described above, by comparison to standards prepared from known mixes of colloidal and agglomerated stocks. As can be seen from Table I, every compound tested produced some amount of stable colloidal material. Where noted, stability, following HGMS and resuspension in phosphate buffer, is also indicated. In all cases the success of the coating experiment could be determined immediately following the sonication procedure from light scattered or by the apparent "shininess" of the solution. Materials for which coating conditions were inappropriate (i.e., stable coated subdivided particles were not produced) were by contrast dull and heterogeneous in appearance.

**TABLE I**

| **Supernatant Coating Material and Concentration (wt %)** | **Percent Colloidal** | **HGMS Recovery** | **Treatment of Colloidal Subsequent Resuspension** |
|---|---|---|---|
| Dextran, 25% | 100% | Good | Good |
| Dextran, 12.5% | 50% | Good | Good |
| Dextran, 6.25% | 25% | ND | ND |
| Dextran, 3.12% | 5% | ND | ND |
| | | | |
| SDS, .5% | 5% | ND | ND |
| SDS, .25% | 20% | ND | ND |
| SDS, .125% | 80% | Good | Poor |
| SDS, .063% | 50% | ND | ND |
| | | | |
| Tween-20, 1% | 20% | Good | Fair |
| Tween-20, .5% | 30% | ND | ND |
| Tween-20, .25% | 40% | ND | ND |
| Tween-20, .125% | 50% | ND | ND |
| Tween-20, .063% | 75% | ND | ND |
| Tween-20, .031% | 90% | ND | ND |
| Tween-20, 3.5% | 50% | ND | ND |
| | | | |
| BSA, 1% ; 50mM P | 95% | Good | Good |
| BSA, 7.5%; 50mM P | 80% | ND | ND |
| BSA, 7.5%; 40mM P | 75% | ND | ND |
| BSA, 7.5%; 30mM P | 50% | ND | ND |
| BSA, 7.5%; 20mM P | 90% | ND | ND |
| BSA, .25%; | | | |
| .016% Tween-20 | 95% | Good | Good |
| | | | |
| IgG(human), 5%; | | | |
| PBS, 50% | 60% | Good | Good |
| | | | |
| Lipid Stripped | | | |
| Human Sera | 100% | Good | Good |
| | | | |
| G α MFc/50mM P 1:2 | 5% | ND | ND |
| G α MFc/50mM P 1:4 | 50% | ND | ND |
| G α MFc/50mM P 1:8 | 5% | ND | ND |
| G α MFc/50mM P 1:16 | 30% | ND | ND |
| G α MFc/50mM P 1:32 | 20% | ND | ND |
| G α MFc/50mM P 1:64 | 5% | ND | ND |
| | | | |
| Steroid Free, Lipid | | | |
| Stripped Sera, Neat | 5% | ND | ND |
| | | | |
| Steroid Free, Lipid | | | |
| Stripped Sera/50mM p | | | |
| (1:128) | 50% | ND | ND |
| PEG, 20% | 80% | ND | ND |
| GLA, 10% | 100% | ND | ND |
| poly G, 10% | 15% | ND | ND |
| polyvinyl | | | |
| pyrrolidone, 15% | 75% | ND | ND |
| polyvinyl | | | |
| alcohol, 10% | 50% | ND | ND |
| - ND =: Not Done - p =: Phosphate buffer - G α Mfc =: goat anti-mouse Fc [Jackson Labs, West Grove, PA] - SDS =: sodium dodecyl sulfate - PEG =: polyethylene glycol [Matheson, Coleman and Bell, East Rutherford, NJ] - Dextran =: Dextran T-40 [Pharmacia, Piscataway, NJ] - poly G =: polyglutamic acid [NEN, Pilot Chemical Division, Boston, MA] - GLA =: [(glutamic acid 45 mole%) (lysine 35 mole% (alanine 20 mole%)]ₙ [NEN, Pilot Chemical Division, Boston, MA] - IgG =: Immunoglobulin G Lipid stripped and steroid free lipid stripped sera from Scantibodies Inc., Santee, - CA Tween-20 =: Polyoxyethylene surfactant (ICI Americas) | | | |

### COMPARATIVE EXAMPLE 2

### Quantitation of Protein Coating and Retention

1-125 labeled BSA and IgG were prepared by the Iodogen method of Fracker and Speck (Biochem. Biophys. Res. Comm. 80 849, (1978)). Specific activities were 520,000 cpm/µg and 810,000 cpm/µg, respectively.

BSA was coated onto magnetite under two sets of conditions as follows (1) at 7.5% BSA in 10 mM phosphate buffer followed by 3 minute sonication and (2) at 7.5% BSA in 25 mM phosphate buffer followed by 3 minute sonication. In both cases 800,000 cpm of labeled BSA was added to the mixture (0.5 ml) prior to sonication. From visual inspection it was apparent that the higher concentration of phosphate resulted in significantly more colloidal material. The magnetically collectable precipitate for each experiment was collected, washed twice in 20mM phosphate buffer and counted. The magnetic agglomerated material of conditions 1 and 2 contained 2421 and 2828 cpm, respectively. To determine how well BSA had adsorbed to these agglomerated preparations the recovered material was suspended in 0.1 M glycine at pH 3.0 for 40 minutes at room temperature, magnetically separated and washed once with 20mM phosphate buffer. For experimental conditions 1 and 2, counts retained were respectively 91 and 84%. When similar preparations were resuspended in buffer and allowed to incubate over night at 37°C, no material desorbed from the magnetite.

To determine the stability of the colloidal magnetite BSA, the amount of BSA adsorbed onto the colloid was quantified as follows: 3x100 µl aliquots of the supernatant were individually collected by HGMS, washed two times with 300 µl of phosphate buffer and resuspended into 100 µl of the same buffer. By observation over time (16 hours) it was apparent that the colloidal material was stable. Radioactive BSA recovered by HGMS for the total supernatant was 5400 cpm. From radioactivities and volumes of the supernatant and agglomerated materials obtained for condition 2 it was determined that 7,828 cpm's had been incorporated onto magnetite. This amounts to 0.01% of the total BSA added to the system which corresponds to about 0.75 mg/ml of magnetite so prepared. This value is very close to the optimal amount of BSA which can be coated onto magnetite by the procedure of Owen et al.

The IgG coating experiments were done at 5% protein concentration in PBS/2 (a 1:2 dilution of PBS in H₂O). Before sonication, mixtures were spiked with 1.8 x 10⁶ cpm of radio labeled IgG. For these experiments it was found that 1.2% of the total added protein was adsorbed which corresponds to 1.2mg IgG/ml of magnetite. Again this is close to the maximum coating obtainable by the Owen et. al. teaching. When HGMS was performed on the magnetic supernatant of this experiment 23,000 cpm were retained on the colloidal material. The agglomerated magnetic pellet retained 17,300 cpm's after multiple washings. When the magnetic pellet was resuspended in glycine at pH 3.0 as above, only 50% of the counts were retained on the subsequent magnetic pellet. However, in these experiments it was found that glycine treatment of the microagglomerated material had converted a substantial portion (approximately half) to colloidal material; hence, the IgG coated material is indeed stable. Colloidal materials recovered by HGMS were stable by visual observation.

### COMPARATIVE EXAMPLE 3

### Retention of Biological Activity

Goat anti-mouse Fc (obtained from Jackson Laboratories, West Grove, PA) was coated onto magnetite as described in Comparative Example 1. For coating, the untreated anti-sera was diluted 1:4 with 50 mM phosphate. After the sonication procedure, most of the resultant material appeared colloidal. The entire supernatant was separated on Sephacryl-300. Colloidal material appearing in the void volume was recovered and the following test performed: 100 ul of the recovered colloid was mixed with either 100,000 counts of 125-I labeled mouse IgG or 100,000 counts of 125-I BSA. These mixtures were incubated in 12 x 75 mm tubes in the presence of iron powder as described above. After 90 minutes at room temperature, HGMS was performed, as described above, supernatants were discarded, and collected material washed twice with 0.8 mls of PBS containing 2% BSA. For these colloid samples (in triplicate) it was found that, on average, 5,200 counts of mouse IgG was bound to the Fc-coated colloid versus 632 cpm of nonspecifically bound BSA.

### COMPARATIVE EXAMPLE 4

### Light Colored Particles

Mixed transition metal oxides were prepared at room temperature and at 65°C by addition of base to appropriate metal chlorides as described in Comparative Example 1. Table II lists preparative conditions, molar ratios, initial color of the oxides and color after 1 week, or color after bubbling O₂ through freshly prepared oxides for eight hours.

**TABLE II**

| **Concentration (mMole/Liter)** | | | | | | | | |
|---|---|---|---|---|---|---|---|---|
| Part. | FeCl₂ | VCl₃ | CoCl₂ | PdCl₃ | DyCl₃ | Original ErCl₃ | Final Color | Color* |
| 1 | --- | 5 | --- | --- | 5 | --- | dk green | white |
| 2 | 2.7 | 3.6 | --- | --- | 3.6 | --- | dk green | yellow |
| 3 | 5 | 3.3 | --- | --- | 1.7 | --- | dk green | orange |
| 4 | 6 | --- | --- | --- | 4 | --- | dk green | rust |
| 5 | --- | --- | 5 | 5 | --- | --- | green | brown |
| 6 | --- | --- | --- | 5 | 5 | --- | brown | brown |
| 7 | --- | 5 | 5 | --- | --- | --- | blue/green | green |
| 8 | --- | 5 | --- | --- | --- | 5 | It brown | white |
| 9 | --- | --- | --- | 5 | --- | 5 | brown | brown |
| 10 | --- | --- | --- | --- | 5 | 5 | white | white |
| 11 | 5 | --- | --- | --- | --- | 5 | green | orange |

| | | | | | | | | |
|---|---|---|---|---|---|---|---|---|
| *These oxides change color gradually in about one week, or in about eight hours if the new precipitate is bubbled through with oxygen. | | | | | | | | |

Table III lists the results of coating the oxide preparations of Table II (preps 1, 2, 3 and 4) with dextran and with BSA, according to the method of this invention. Clearly these materials can be coated similarly to magnetite. It is noted that the non-ferrite and ferrite oxides listed in Table II could be used in place of magnetite with the teaching of Whitehead et al. to produce similarly colored silane coated materials. When the appropriate chlorides (or sulfates) of these metals are used with the teachings of Molday or with Owen et al., it is possible to obtain colloidal materials which are nearly transparent in the visible region.

**TABLE III**

| **Appearance After Sonication** | | | | |
|---|---|---|---|---|
| **Particle** | **Compound** | **Supernatant** | **Pellet** | **% Colloidal** |
| 1 | 25% Dextran | Shiny White | White | 90% |
| 2 | 25% Dextran | Cloudy Yellow | White | 20% |
| 3 | 25% Dextran | Clear Yellow | White | 95% |
| 4 | 25% Dextran | Clear Orange | Orange | 50% |
| 1 | 1% BSA/50mM P | Shiny White | White | 90% |
| 2 | 1% BSA/50mM P | Cloudy Yellow | White | 90% |
| 3 | 1% BSA/50mM P | Clear Yellow | White | 95% |
| 4 | 1% BSA/50mM P | Clear Orange | Orange | 75% |

### COMPARATIVE EXAMPLE 5

### Colloid Particle Size

BSA magnetite was prepared with the magnetite of Comparative Example 1 and BSA at 1% in 50mM Phosphate at pH 7.0 as described above except that an aliquot was sonicated for a second three minute period and another for a third three minute period. After removal of any agglomerated material with the magnetic rack, as described above, the resultant colloidal samples were sized by laser light scattering (Coulter Submicron Particle Analyzer). Within experimental error each preparation had a mean particle diameter of 80nm.

Next, magnetite for coating was prepared by two methods which have been found to decrease particle size in the Owen et al. procedure, namely very rapid addition of base and very rapid addition of base at elevated temperature (65°C). When BSA magnetite was prepared with a single 3 minute sonication step but with magnetite prepared according to Owen et al., the resultant colloids were both of 50nm mean diameter.

### EXAMPLE 1

### Properties of Magnetite as Affected by pH

3.75% NH₄OH was added at 0.6 mls/minute to a 200 ml stirred mixture of degassed hydrated ferric and ferrous chloride salts at 7 and 3 mg/ml respectively. As the pH of the initially acidic mixture approached 7.0 (indicted by a color transition from dark orange to black), 300ul aliquots were removed, placed in 10x75 glass test tubes, and tested for the presence of magnetite, by placing a neodymium/iron/boron bar magnet to the side of the tube and visually observing magnetic clearing. The transition from non-magnetic or partially magnetic material to totally magnetic material occurred at pH 7.4 after 12.2 mls of base had been added. At this point, 60 ml of the mixture was removed and designated preparation A. Additional base was added to the mixture to pH 8.9; a 60 ml aliquot was removed and labelled preparation B. Base was further added to the reaction mixture to pH 9.8 and an aliquot of this material designated preparation C. Magnetite preparations A, B, and C were washed 4 times with distilled water and resuspended in water such that the iron salts were at the starting concentrations of the original solution. 0.5 ml aliquots of the latter suspensions of A, B and C were sonicated for 3 minutes at 70% output (Fisher Sonic Dismembrator). Immediately following sonication, each had the shiny appearance of colloidal magnetite; however, all reverted to dull suspensions (indicative of particle aggregates) within 2 minutes. For preparation C, the transition occurred 20-30 seconds following sonication; for preparation B, 40-50 seconds; while preparation A required 2 minutes for this transition. When the preparations were placed in the Corning magnetic rack immediately following sonication, they cleared completely from suspension in the same order as above and over the same time frame. After reaggregation, there was no discernible difference among the samples.

To determine if prepared magnetites' susceptibility to dissociation by sonication is altered by H⁺ or OH⁻ treatment, 0.5 ml aliquots of the preparation C suspension were placed in test tubes and separated from their water supernatants by magnetic separation and supernatant aspiration. Magnetic pellets were then resuspended into 0.5 ml of either dilute HCl (0.1, 0.01, 0.001, or 0.0001 M) or dilute NaOH (0.1, 0.01, 0.001, or 0.0001 M), sonicated for 3 minutes as above and visually inspected. For these samples, it was apparent that acid or base treatment increased the disruption of magnetite in proportion to concentration of acid or base. The aliquots resuspended and sonicated in 0.1 M HCl or NaOH remained shiny, i.e. colloidal, for nearly 12 hours. The aliquots resuspended in 0.001 M acid or base also exhibited colloidal behavior for significant periods of time. On the other hand, aliquots resuspended into 0.0001 M acid or base quickly aggregated after sonication much like the parent material did in water.

### COMPARATIVE EXAMPLE 6

### Coating of pH Modified Magnetite

Preparation C of Example 1 was coated by disruption via sonication with anionic and cationic polypeptides. An anionic terpolypeptide composed of 60 mole % glutamic acid, 30 mole % alanine, 10 mole % tyrosine (GAT, Lot #M18G) and a cationic copolymer of 60 mole % lysine, 40 mole % alanine (LA, Lot #M-5B), both obtained from Pilot Chemicals, Watertown, MA, were used. These polypeptides, both of about 100,000 Daltons, were solubilized by trituration with appropriate acid or base, neutralized, dialyzed versus phosphate buffered saline (pH 7.0) and subsequently against distilled water. Coatings were attempted with 10 mg/ml solutions of polypeptide and serial dilution of same to 1/16. Coating experiments were done by sonication as described above. For these experiments, the 1/16 and 1/8 dilutions of both GAT and LA produced stable colloidal solutions. The 1/4, 1/2 and undiluted polypeptide solutions resulted in distributions of colloid and agglomerated material. The amount of colloid both instances generally decreased with increasing polypeptide concentration. However, at 25 mg/ml GAT all of the material was colloidal.

Preparations A and C of Example 1 were coated with GAT. 0.5 ml aliquots of magnetite were resuspended in 1.0 ml of HCl (0.01 and 0.1 M) or NaOH (0.01 and 0.1M) for 60 seconds followed by two 0.5 ml water washes. These pre-treated magnetites were resuspended in 0.5 ml aliquots of 1 mg/ml GAT containing 2.5 x 10⁶cpm ^{125I}GAT (radio-labeled by the Iodogen method described above) and sonicated as described. Sonicated samples were placed in the Corning magnetic rack and left to separate overnight. Magnetic pellets were washed once in 0.5. M NaCl and counted. Counts bound and percentages of GAT coated onto the various magnetites are given in Table IV.

**TABLE IV**

| Effect of H⁺/OH⁻ Treatment of Magnetite Preparations A and C on GAT Coating | | | | |
|---|---|---|---|---|
| Treatment | Preparation A | | Preparation C | |
| | cpm bound | % bound | cpm bound | %bound |
| 0.10 M HCl | 450,000 | 35 | 340,950 | 26 |
| 0.01 M HCl | 287,602 | 22 | 226,250 | 17 |
| H₂O Control | 209,165 | 16 | 236,042 | 18 |
| 0.01 M NaOH | 130,728 | 10 | N.D. | N.D. |
| 0.010 M NaOH | 90,600 | 8 | 54,010 | 5 |
| N.D. = Not done | | | | |

From the data of Table IV, it is clear that magnetite crystal surface charge can be made more positive by a pre-treatment with acid and conversely, treatment with base generally reduces sites available to the negatively charged polymer. When ¹²⁵I preparation of the cationic terpolymer LAT (60 mole % lys, 30 mole % ala, 10 mole % tyr), (received from Dr. H.J. Callahan, Jefferson Medical College, Philadelphia, PA) was used with similarly pre-treated magnetite, it was observed that greater binding of this positively charged material occurred on magnetite pre-treated with base.

To determine if crystal surface charge alteration via acid or base treatment is a general property of transition element oxides, such as those prepared from different molar ratios of chlorides of Fe (II), Dy (III), V (III), such materials were treated with acid or base as above and mixed with either radio-labelled GAT and/or Salmon sperm DNA (Sigma Chemical Co., St. Louis, MO). As in the case with magnetite, acid treatment promoted binding of these negatively charged polyelectrolytes. In fact, uncoated materials pre-treated with 0.1 M HCl, followed by water wash on sonication give materials which behaved like preparations of magnetite described above, i.e., transiently stable colloids. When these materials were in this semi-stable colloid state, addition of Salmon sperm DNA in appropriate quantities led to complete agglutination indicating the crystals had assumed substantial positive surface charge.

### EXAMPLE 2

### Post Sonication Direct Coating

Magnetic ferrofluid was prepared by the addition of coating material after sonication of the bare magnetite according to the procedure described below.

The bare magnetite was prepared by the process as described in Comparative Example 1, above. The magnetite was separated from water by a bar magnet and resuspended in phosphate buffer of pH 7.5. The average particle size of the magnetite was reduced by pulsed sonication (Fisher's Sonic Dismenbrator 550) for five minutes, using 10 second pulses and 10 second pauses in succession.

Immediately following sonication, the first coating material, i.e., 1 mg/ml 9x biotin-BSA in 20 mM phosphate buffer at pH 7.5, was incubated with the sonicated bare magnetite for a period of 15 minutes at room temperature. The second coating material, i.e., 5 mg/ml BSA in 20 mM phosphate buffer at pH 7.5, was added to the mixture of magnetite and first coating material.

Table V shows the effect of varying coating concentrations on the colloidal magnetic particle size over time and the separation rates in a high gradient magnetic field. The particle size was measured by Coulter Sub-micron Particle Analyzer (Model N4SD). The magnetic properties of the colloidal particles were determined by high gradient magnetic separation in a 300 µl microtiter well with grids in a 4 kG uniform magnetic field.

**TABLE V**

| **Effect of concentration of Primary and Secondary Coatings on Stability and Separation Rate of Colloidal Magnetic Particles** | | | | | | |
|---|---|---|---|---|---|---|
| [1° Coating] (mg/ml) | [2° Coating] (mg/ml) | Particle Size* (nm) | | | % Separation | |
| | | 0 | 24 | 168 (hrs) | 30 | 60(sec) |
| 0.0 | 5 | 106 | 66 | 68 | 70 | 90 |
| 0.1 | 5 | 74 | 83 | 86 | 80 | >90 |
| 0.3 | 5 | 77 | 81 | 83 | 81 | >93 |
| 0.5 | 5 | 76 | 83 | 85 | 85 | >95 |
| 0.7 | 5 | 78 | 83 | 82 | 88 | >95 |

| | | | | | | |
|---|---|---|---|---|---|---|
| * For the duration of the stability test, all colloidal magnetic particles remained clear and in suspension. | | | | | | |

### COMPARATIVE EXAMPLE 7

### Preparation of Stable, Colloidal Magnetic Particles by Direct Coating of Biotinylated BSA with Simultaneous Sonication

Stable, colloidal magnetic particles were prepared by simultaneous size reduction of particulate magnetic starting material and coating during sonication.

The bare magnetite was prepared by the process as described in Comparative Example 1, above. The magnetite was separated from water by a bar magnet and resuspended in 1 mg/ml 9x biotin-BSA in 20 mM phosphate buffer of pH 7.5. The average particle size of the magnetite was reduced by pulsed sonication (Fisher's Sonic Dismenbrator 550) for one minute, using 10 second pulses and 10 second pauses in succession.

The particle size and the magnetic separation rate was measured as described in Example 2, above.

The average particle size was 80 nm. The separation rate was 85% in 30 seconds and >95% in one minute. The resultant product was monitored over a period of two weeks and was found to be stable, as the direct coated magnetite remained in suspension and was clear in appearance.

### COMPARATIVE EXAMPLE 8

### Binding and Removal of DNA From Solution Using Uncoated Colloidal Magnetic Particles

This example shows that pure λ DNA can be bound and recovered using bare, uncoated colloidal magnetic particles under acidic conditions.

The colloidal magnetic particles were prepared by magnetically sedimenting suspensions of bare magnetite prepared as in Comparative Example 1, in water at a level of 1 mg iron/ml solution. The magnatant (non-sedimented liquid phase) was aspirated and resuspended in 0.1 M NaCl to a volume equal to the original. The suspension was vortexed for five seconds and then washed three times successively with deionized water. During each wash, the magnetite was held in place magnetically.

Two test samples of DNA fluorescently labelled with Hoechst dye H33258 (LIFE TECHNOLOGIES catalogue # 5250SB) were prepared. The first sample, Sample A, was made up by adding 1 ml of 8 µg/ml λ DNA in 20 mM phosphate buffer at pH 5 immediately to a first test tube with agitation. The resultant mixture was then sonicated in the tube on ice using microtip probe for one minute (Fisher Sonic Dismembrator, model 550; setting 3, pulse cycle 1 second on/l second off). The second sample, Sample B, was subjected to the same protocol, except that one ml of 8 µg/ml λ DNA in 20 mM phosphate buffer at pH 7.5 was used.

Amount of DNA was assayed by fluorometry, by comparing the amount of fluorescence before and after magnetic separation. Table VI shows the fluorescent signals and calculated % DNA removed from solution by binding to the colloidal, magnetic particles.

**TABLE VI**

| **Effect of pH on DNA Extraction from Solution** | | | | |
|---|---|---|---|---|
| Sample | pH | Fluorescence before M.S. | Fluorescence after M.S. | % DNA Capture |
| A | 5 | 60 | 29 | 52 |
| B | 7.5 | 60 | 63 | 0 |

### COMPARATIVE EXAMPLE 9

### Binding of DNA by Bare Magnetite and Recovery via Buffer Solution

This example shows that λ DNA captured under acidic conditions can be removed from bound colloidal magnetic particles by the selection of the wash buffer.

The magnetite and λ DNA were prepared as described in Comparative Example 8, above. Ten samples of the magnetite were prepared. All were sonicated with fluorescently labelled DNA under acidic conditions at pH 4 using 20 mM phosphate buffer. Initial fluorescent signal for the starting solution of DNA was 80. The DNA was magnetically pulled for 10 minutes and the supernatant was measured. From the difference between the starting fluorescence and the supernatant signal, the % DNA recovered was calculated.

Table VII shows that the percent extracted by sonication varied between 58 to 63%. The relative efficiencies of the wash buffers is shown as % recovery.

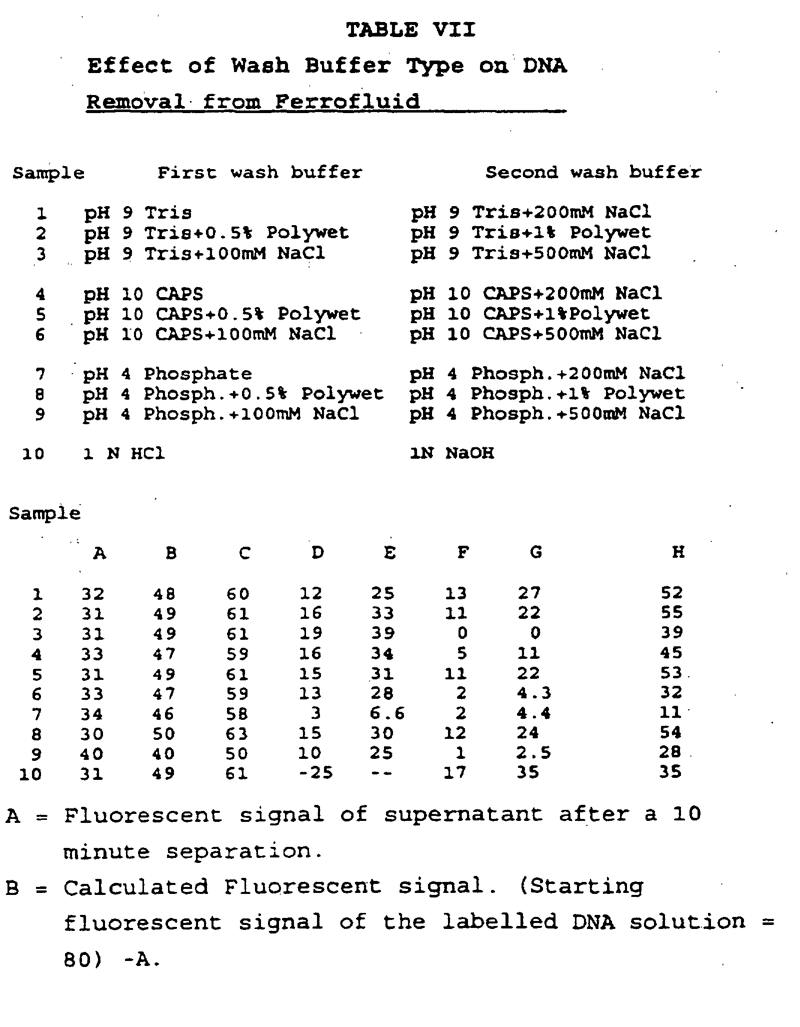

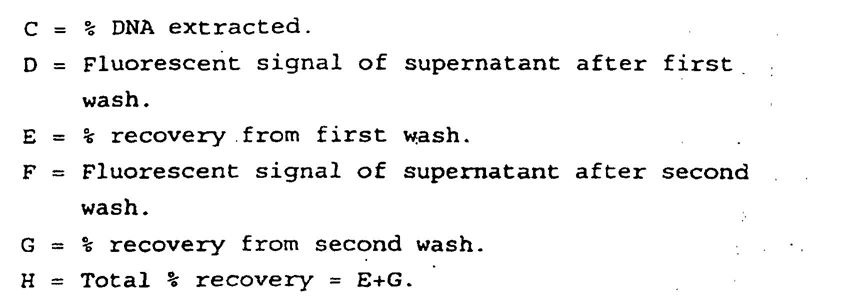

All reagents and chemicals used in examples 12-23 were of analytical grade and obtained from Fisher Scientific (Pittsburgh, PA) unless otherwise specified.

### EXAMPLE 3

### Hot Sonicated Magnetite Followed by Hot Coating Reaction

Magnetite was prepared by mixing solutions of 17 g and 12 g of ferric sulfate pentahydrate and ferrous sulfate heptahydrate, respectively, in water with stirring at 70°C under a nitrogen atmosphere while raising the pH with 60 ml of ammonium hydroxide. The resultant magnetite was collected magnetically, washed 10 times with distilled water and resuspended in 600 ml distilled water. The preparation so made contained approx. 10 mg/ml magnetite.

To make bovine serum albumin (BSA)-ferrofluid, 1.0 gram of the magnetite prepared above was measured into a beaker and magnetically washed with water two times. The final resuspension was into 100 ml of 20 mM sodium phosphate, pH 7.5. The magnetite was preheated to 70°C, then sonicated with a Fisher Sonic Dismembrator Model 550 for 20 minutes with pulse sonication 1 second on/l second off (total time 40 min.) at a power setting of 7. Meanwhile, 1.8 g of BSA was dissolved in 60 ml of 20 mM sodium phosphate, pH 7.5, and heated for 10 min. at 75°C. After sonication, 30 ml of the sonicated magnetite was quickly removed, mixed with the hot BSA and heated for 5-20 minutes at 75°C, then cooled in an ice bath. The ferrofluid size distribution was narrowed by a series of controlled magnetic washes in a chamber having a 3 kGauss field at its surface. Material collected in a fixed time interval was retained. Routinely, this procedure was done three times for each preparation. Hereinafter this process is referred to as high field washes. Values for size, salt stability, and adsorbed protein as measured by carbon analysis are tabulated in Table VIII, rows 1-3.

### EXAMPLE 4

### Cold Sonicated Magnetite Followed by Hot Coating Reaction

Magnetite was prepared as described in example 3 above. To make BSA ferrofluid, 3.6 grams of BSA was dissolved in 120 ml of 20 mM sodium phosphate, pH 7.5, and heated for 10 min. at 75°C. Meanwhile, 1.0 gram of the magnetite prepared above was measured into a beaker and magnetically washed with water two times. The final resuspension was into 100 ml of 20 mM sodium phosphate, pH 7.5. The magnetite was sonicated with a Fisher Sonic Dismembrator Model 550 for 30 minutes at 10°C with pulse sonication 1 second on/1 second off (total time 60 min.) at a power setting of 7. The sonication temperature was controlled with a circulating cooling system containing ethylene glycol at -4°C. After sonication, 60 ml of the sonicated magnetite was quickly removed and mixed with the hot BSA and heated for 5-60 minutes at 75°C, then cooled in an ice bath. The ferrofluid was washed in the high field. Measurements of size, salt stability, and adsorbed carbon are tabulated in Table VIII, rows 4-9.

### EXAMPLE 5

### Measurement of Salt Stability and Adsorbed Protein of Ferrofluid

To measure the salt stability of the preparation of BSA ferrofluid, 0.25 ml samples of the ferrofluid were placed in NaCl-containing phosphate buffer such that the final concentrations were 0, 0.5, 1.0, and sometimes 2.0 M in sodium chloride. The samples were then sized with a Coulter N4CD Submicron Particle Analyzer (Coulter Corp., Hialeah, FL) at t = 0, 1, 2, 4, and 17 hours at room temperature.

To measure the amount of adsorbed protein, 2-3 mg of ferrofluid was mixed with 1 ml concentrated HCl, placed in a glass ampoule, and sealed. After overnight digestion at 110°C, the sample was neutralized to a pH of 2-4. The total adsorbed carbon was measured with a TOC 5000 (Shimadzu, Kyoto, Japan.) The results of the above procedure are summarized for the ferrofluids prepared in examples 3 & 4 in Table VIII, below. As a control, one sample of ferrofluid (cold sonicated magnetite as described in example 3) was mixed with the BSA and left unheated at room temperature (20°C) for 30 minutes, see row 10.

**TABLE VIII**

| Row | Sonication conditions | Post heat time with BSA | Size (nm) | Salt stability: followed by change in size (nm) after 17 hours © RT. | | | TOC (µg BSA/ mgFe) |
|---|---|---|---|---|---|---|---|
| | | | | 0 M | 0.5 M | 1.0 M | |
| 1 | Sonicating BM alone, hot for 20min followed by post heating with BSA. | 5 min. | 146 | 144 | 170 | 500 | 255 |
| 2 | | 10min | 139 | 144 | 148 | 194 | 324.1 |
| 3 | | 20min | 144 | 148 | 148 | 153 | 351 |
| 4 | Sonicating BM alone, cold for 30 min followed by post heating with BSA. | 5 min. | 145 | 157 | 683 | 1985 | 278 |
| 5 | | 10min | 145 | 154 | 350 | 982 | n.d. |
| 6 | | 20min | 144 | 155 | 151 | 265 | n.d. |
| 7 | | 30min | 145 | 159 | 169 | 337 | n.d. |
| 8 | | 45 min | 149 | 156 | 157 | 188 | 343 |
| 9 | | 60 min | 154 | 165 | 173 | 185 | 359 |
| 10 | Sonicating BM alone, cold for 30 min followed by cold incubation with BSA. | Cold 30 min | 266 | 862 | 1675 | 1510 | 134 |
| n.d. = not determined BM = bare (uncoated) magnetite slurries | | | | | | | |

The margin of error in the size data is approximately 5%, so small changes are within error. However, larger sized changes are significant. A particle above approx. 300 nm will eventually irreversibly settle out of solution. The margin of error in the adsorbed carbon data is approximately 4%. Note that the longer "post heat time" with the BSA results in ferrofluid that are highly coated (>300 ug BSA/mg Fe) with BSA. Also note that the size of these ferrofluid particles remain relatively constant, even at high salt concentration, which satisfies the criteria for salt stability. Finally note that cold control (row 10) has significantly less of a BSA coating, and it is highly unstable in even salt solutions containing only buffer ions (20 mM phosphate).

### Comparative Example 10

### Heat Treatment of Cold Sonicated BSA Ferrofluid

Ferrofluid can be prepared as disclosed in U.S. patent application 397,106, with a cold sonication of a mixture of magnetite and protein. The heating of this ferrofluid will also result in increased protein coating and salt stability. Magnetite was prepared as described in comparative example 1, above. BSA ferrofluid was prepared by mixing 2.0 g of BSA with 1.0 g of magnetite in 200 ml. Then the mixture was sonicated with a Fisher Sonic Dismembrator Model 550 for 45 minutes with pulse sonication 1 second on/l second off (total time 90 min.) at a power setting of 7. The sonication temperature was controlled with a circulating cooling system containing ethylene glycol at -4°C. The measured temperature during the sonication was 30°C. Then the resultant ferrofluid was heated for varying times ranging from 0-90 minutes at 80°C.

The adsorbed protein was measured by the protein assay kit commercially available from the BioRad Corp. (Richmond, CA) The samples were prepared for the assay by removing all magnetic particles from solution with a 5 min HGMS pull in a microtiter well fitted with a wire screen placed in an Immunicon Protein Separator as described in US patent 5,200,084 (Immunicon Corp., Huntingdon Valley, PA). The supernatant was removed from the microtiter well with a pipet, diluted, and the assay was performed as directed in the kit's instructions, using a standard curve prepared from pure BSA. The protein bound to the magnetic particle was determined by subtraction of the amount of BSA found in the non-magnetic supernatant from the original amount of BSA added to the magnetite.

The results of the experiment are displayed on graph I below, in which the amount of BSA adsorbed per mg of iron is plotted as a function of the heating time. The sudden increase of coating that occurs at approximately 30 minutes correlates with an increase in the salt stability (data not shown).

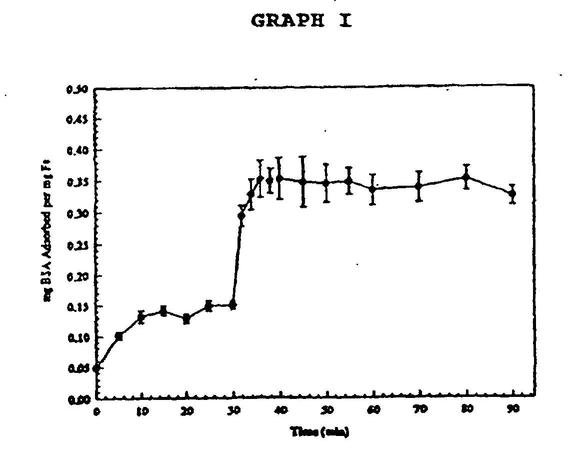

### COMPARATIVE EXAMPLE 11

### Non-specific Binding as a Function of Heating During Sonication Time

Magnetite was prepared as in example 3 above. To make BSA ferrofluid, 0.175 gram of the magnetite was measured into a beaker and magnetically washed with water two times. The final resuspension was into 35 ml of BSA solution prepared at 10 mg/ml in 10 mM sodium phosphate, pH 7.5. The mixture was placed in a jacketed beaker (Heat Systems, Farmingdale, NY) and cooled or warmed to temperatures listed in Table IX below. Then the mixture was sonicated with a Fisher Sonic Dismembrator for 20 minutes with pulse sonication 1 second on/1 second off (total time 40 min.) at a power setting of 7. The actual temperature of the sonicate was measured. After sonication, the BioRad Assay was performed as described in comparative example 10 to determine the amount of bound protein. Then the ferrofluid size distribution was narrowed by a series of 3 magnetic washes with "high field magnets." Resuspension was into 10 mM sodium phosphate, pH 7.5. Then the ferrofluid was further fractionated with two "low field" magnetic washes. The strength of the magnetic field was approximately 0.4 kGauss at the collection surface. In this case, only the supernatant was collected after each wash, and the pellet was discarded.

Size, salt stability, and intrinsic NSB were measured at this point. NSB (non-specific binding) is defined in this case as the percentage of cells removed from solution when the cells are mixed with a ferrofluid, which is then allowed to magnetically collect. In the ferrofluid/cell solution, there is no substance that should cause the ferrofluid and cells to interact. For example, no antibodies, lectins, or common capture agents, such as biotin, streptavidin, haptens, or Protein A or G are present. The NSB was measured with a radioactive difference assay. CEM cells were labeled with ⁵¹Cr by suspending up to 5.0 x 10⁷ cells in 2 ml of RPMI supplemented with 10 % fetal calf serum, 100 units of penicillin-streptomycin, and 1.25% L-glutamine (all supplied by Mediatech, Washington, DC). ⁵¹Cr was obtained from Dupont (Wilmington, DE) and used straight from the bottle. The cpm of the chromium was determined by counting in a Cobra II Gamma Counter (Packard, Downer's Grove, IL). Approximately 1 x 10⁷ cpm's were added to the cells and incubated at 37°C for 1 hour, vortexing every 15 minutes. For the assay, 160 µl of labeled cells at 2.5 x 10⁶ cells/ml were mixed with 160 µl of ferrofluid at 20 µg Fe/ml in isotonic phosphate buffered saline (IPBS) in a test tube. The mixture was incubated for 5 minutes. During the incubation time, the counts of ⁵¹Cr were determined by counting in the gamma counter. Then 250 µl of the mixture was placed into a microtiter well, and the ferrofluid was removed with a 5 minute magnetic depletion in an Immunicon Cell Separator as described in US patent 5,200,084 (Immunicon Corp., Huntingdon Valley, PA). After depletion, the microtiter well(s) were removed and individually placed into test tubes. The number of counts were recorded. Additionally, the incubation mixture remaining in the test tube after the sample was removed to the microtiter well was counted for ⁵¹Cr. The starting number of counts was determined by subtracting these counts remaining in the test tube from the number of counts initially added to each test tube. Percentage removal (NSB) was determined by the following equation:$\text{NSB =} \frac{\text{(100%) (starting counts-counts in microtiter well)}}{\text{(starting counts)}}$ Data for this example are tabulated in Table II below. Note that only above approximately 60°C actual sonication temperature does the ferrofluid become salt stable. At the higher temperatures, the NSB drops as well, which could be caused by the elimination of "bare spots" on the magnetic particle, which due to the positive charge of the iron, may tend to inherently bind to cells, non-specifically removing them from solution.

**TABLE IX**

| Temp (°C) | Actual Sonication Temp (°C) | Adsorbed Protein (µg BSA/mgFe) | Salt stability: followed by change in size (nm) in 0.75M NaCl | | | NSB (%) |
|---|---|---|---|---|---|---|
| | | | 0 hr | 2 hr | 25 hr | |
| -4 | 31 | 73.4 | 91 | settled | settled | 90.7 |
| 10 | 38 | 117 | 93 | settled | settled | 87.7 |
| 25 | 47 | n.d. | 103 | 755 | settled | 55.3 |
| 35 | 55 | n.d. | 107 | 144 | settled | 39.6 |
| 45 | 62 | 196 | 113 | 123 | 194 | 38.7 |
| 55 | 69 | 198 | 119 | 123 | 135 | 25.0 |
| 65 | 75 | n.d. | 126 | 130 | 137 | 19.4 |

### EXAMPLE 6

### Coupling Streptavidin to BSA Ferrofluid

Streptavidin can be coupled to BSA ferrofluid by the following procedure. Hot coated ferrofluid was prepared as in Example 4, with a 60 minute BSA coat-time. The ferrofluid was decanted and washed three times with a high field magnet. After each wash, the ferrofluid was resuspended in 180 ml 0.1 M sodium phosphate, pH 7.5. The BSA ferrofluid was activated using N-succinimidyl-4-(N-maleimido methyl) cylcohexane-1-carboxylate (SMCC) (Pierce, Rockford, IL) following the manufacturer's instructions. Then the activated ferrofluid was washed three times. After each wash, the ferrofluid was resuspended in 180 ml 0.1 M sodium phosphate, pH 6.5 at 4°C. An amount of streptavidin (Prozyme, Richmond, CA) equal to twice the mass of iron was weighed out and dissolved in 0.1 M sodium phosphate, pH 7.5 with 5 mM EDTA. The streptavidin was activated with Trauts reagent (Pierce, Rockford, IL) following the manufacturer's instructions. Then the activated streptavidin was purified over a PD-10 column (Pharmacia Biotech, Uppsala, Sweden) and 1 ml column fractions were collected. Fractions 4 and 5 contained protein and were pooled. The activated ferrofluid and 1.5 mg of activated streptavidin per milligram of iron were then mixed and allowed to react at room temperature for 4 hours with stirring. Then the reaction was quenched with 4 mg/ml mercaptosuccinic acid in 0.1 M sodium phosphate, pH .7.5 with 5 mM EDTA. The quenching reaction was allowed to react at 4°C for 16 hours with stirring. After the quench reaction, the ferrofluid was washed two times with a high field magnet. After each wash, the ferrofluid was resuspended in 150 ml 0.1 M sodium phosphate, pH 7.5 with 0.2 mg/ml BSA. However, the final resuspension was in 10 mM HEPES, pH 7.5 with 10 mg/ml BSA. The resultant ferrofluid was immersed in a Fisher bath sonicator FS-14 for 2 minutes, then washed into 10 mM HEPES, pH 7.5 with 0.1 mg/ml BSA. A final 0.2 micron filtration was performed.

### EXAMPLE 7

### Depletion of CEM Cells with Hot Coated Streptavidin Ferrofluid

First anti-CD45 (Becton-Dickinson, San Jose, CA) monoclonal was biotinylated through available free amino groups. Approximately 1-2 mg of antibody was prepared in approximately 0.5 ml of 0.05 M sodium bicarbonate, pH 8.5. N-succinimidyl 6-(biotinamido) hexanoate ester (Molecular Probes, Eugene, OR) was dissolved in DMSO and added to the anti-CD45 in excess. The mixture was allowed to react for 2 hours at 4°C. The antibody was purified over a PD-10 column (Pharmacia Biotech, Uppsala, Sweden), taking fractions 3 and 4 (1 ml fractions.)

CEM cells were harvested and suspended in isotonic phosphate buffered saline solution with 1% BSA (1% BSA/IPBS) to a concentration of approximately 2.2 x 10⁷ cells/ml. A series of 0.85 ml samples of cell suspension were incubated for 10 minutes at room temperature with 1 ug biotinylated anti-CD45. Solutions of streptavidin ferrofluid prepared as in example 6 (lot 188-143-6) and as generally described in US patent application Serial No. 397,106 (lot 0994-1282W) were then added to the cells in a volume of 0.85 ml. The amount of ferrofluid varied from 12.5 ug to 100 ug of iron. The mixtures were allowed to incubate for 5 minutes at room temperature. Then each sample was pipetted into a 2 ml cell separation chamber as described in US patent 5,200,084 (Immunicon Corp., Huntingdon Valley, PA). The samples were allowed to magnetically collect for 7 minutes, then they were removed from the magnetic field. Each sample was then mixed with a pipet and placed again in the magnetic field for another 7 minute magnetic collection, using a fresh set of pins. Efficacy of depletion was determined by counting cell number with a hemacytometer (Hausser Scientific, Horsham, PA) using an ethidium bromide/acridine orange dye, prepared as directed in the BD monoclonal antibody sourcebook and mixed 1:1 with the cell suspension. Results of the depletions are tabulated in Table X below. Note that although both ferrofluids removed the cells efficiently, the hot coated streptavidin ferrofluid removed over 99% of the cells, even at an iron level half of that required by the non-heat treated ferrofluid.

**TABLE X**

| | **Depletion of CEM cells (%)** | |
|---|---|---|
| Total Fe/test | FF lot 188-143-6 | FF lot # 0994-1282W |
| 12.5 ug Fe | 99.4% | 88.5 % |
| 25.0 ug Fe | 99.3% | 98.9% |
| 50.0 ug Fe | 99.5% | 99.3% |
| 75.0 ug Fe | 99.8% | 99.4% |
| 100.0 ug Fe | 99.3% | 99.5% |

### EXAMPLE 8

### Comparison of a Hot Coated Ferrofluid with a Non-Heat Treated Ferrofluid

A hot coated ferrofluid was prepared as described in example 4 and coated with streptavidin as described in example 6 (lot #188-191-15). A non-heat-treated streptavidin ferrofluid was prepared similarly to the ferrofluid used in example 7 (lot #0395-1308). As measured by the TOC 5000, the total adsorbed carbon was 278 µg/mg Fe for the hot coated ferrofluid. For a non-heat-treated ferrofluid, the total adsorbed carbon is approx. 145. These measurements were taken on the BSA particle, before any streptavidin coupling. Additionally the hot coated ferrofluid was salt stable, while the non-heat-treated ferrofluid was not.

However, the difference in protein coating and intrinsic behavior continues after the BSA particle is coated with a secondary protein, such as streptavidin. For example, the binding capacity measures the amount of biotinylated protein that can be bound to a streptavidin ferrofluid. The value for binding capacity is closely related to protein coating, as the more streptavidin coats a particle, the more biotinylated BSA (bBSA) it can bind. The assay for binding capacity began with the labeling of biotin BSA with ¹²⁵I. Biotin-BSA was biotinylated with N-succinimidyl 6-(biotinamido) hexanoate ester (Molecular Probes, Eugene, OR) following the manufacturer's suggested protocol. It was then purified by running it over a PD-10 column, and diluted to 5 mg/ml with 0.25 M sodium phosphate buffer, pH 7.5. 200 µl of Iodogen (Sigma, St. Louis, MO) was placed in a test tube and dried by blowing nitrogen gas over it. One millicurie of ¹²⁵I was added to the test tube, followed by 200 µl of the biotin-BSA. The mixture was incubated on ice for 10 minutes. Then 800 µl of the phosphate buffer was added to the tube and the entire volume was loaded onto a fresh PD-10 column. Labeled biotin-BSA was eluted in the fourth and fifth one ml fraction.

The binding capacity assay was begun with preparation of the standards. Standards between 15 and 500 µg bBSA/ml were prepared with 2.5% ¹²⁵I labeled biotinylated BSA in a phosphate buffer (20 mM phosphate buffer, pH 7.5, containing 10 mg/ml BSA and 0.15 M NaCl). The ferrofluid was diluted to 400 µg/ml with 20 mM HEPES with 0.1 mg/ml BSA and 0.05% ProClin 300 (Supelco, Inc., Bellefonte, PA), pH 7.5. Then the ferrofluid was further diluted ten-fold with the proprietary phosphate buffer noted above and incubated 15 minutes. 100 µl of ferrofluid was placed in each well of a strip of microtiter wells. 100 µl of each standard was then added to each well and allowed to incubate 10 minutes. Then each well was placed in a quadrupole magnetic separator, as described in U.S. Patent 5,186,827, with an opening exactly the size of the microtiter well. After 5 minutes, the non-magnetic supernatant was discarded from each well and the magnetic material was washed five times using PBS buffer with 0.1% Tween 20 with a final resuspension in 20 mM HEPES with 1 mg/ml BSA and 0.05% ProClin 300. Then the wells were removed from the magnetic separator and each well was placed in a 12 x 75 mm test tube. The cpm remaining in each microtiter well were counted with a gamma counter. Additionally, 10 ul of the 500 µg/ml standard (or 5 ug bBSA) was counted in the gamma counter. This number was divided by 1.25 to normalize to 4 µg of bBSA. All sample counts were then divided by this factor and multiplied by 1000 to calculate the number of micrograms of biotin BSA bound per milligram of iron. Results of this binding capacity assay are tabulated in Table XI below for the hot coated ferrofluid (lot#188-191-15) and the non-heat-treated ferrofluid (lot# 0395-1308.) The data indicate that for almost the entire range of bBSA, the ferrofluid manufactured through the hot coated process had approximately twice the binding capacity of a ferrofluid that had not been heat-treated. That is, significantly more biotinylated protein could be bound by the ferrofluid, once the particle had been coated with streptavidin.

**TABLE XI**

| | **ug bBSA/mg Fe** | |
|---|---|---|
| ug bBSA/test | FF lot 188-191-15 | FF lot 0395-1308 |
| 0 | 0 | 0 |
| 1.56 | 15 | 29 |
| 3.13 | 67 | 43 |
| 6.25 | 91 | 53 |
| 12.5 | 96 | 62 |
| 25.0 | 121 | 70 |
| 37.5 | 131 | 71 |
| 50.0 | 120 | 64 |

Another result of the amount of streptavidin bound to a ferrofluid particle is the performance of the ferrofluid in removing cells labeled with biotinylated antibody from solution. Particles with more streptavidin bound can remove more cells at lower amounts of iron. An assay for performance uses nonradioactive CEM cells at 1.0 x 10⁷ cells/ml. 2 ml of cells were mixed with 2.0 µg of biotinylated anti-CD45, prepared as described in example 18. The cells and antibody were incubated for 30 minutes. Then 150 µl of the cell mixture was placed into each of a strip of microtiter wells and then mixed with 150 µl of ferrofluid at 1.5-25 µg/ml. The ferrofluid had been pre-incubated with a blocking buffer (Ferrofluid Dilution Buffer, available from Immunicon Corporation, Huntingdon Valley, PA) for at least 30 minutes. A ten minute incubation was followed by a 5 minute separation in the Immunicon Cell Separator as described in example 18. After removal from the Cell Separator, the supernatant in the wells was mixed with a pipet and 100 µl of sample were removed to a cell counting vial filled with 10 ml of Isotonic Hematall Diluent. The cell numbers were measured with a Coulter ZF Cell Counter (Coulter, Hialeah, FL). Depletion was measured by percentage of cells removed from the sample compared to a sample which had buffer added in place of ferrofluid. Depletions at various iron concentrations are tabulated in Table XII below. It should be noted that although at high iron values, both ferrofluids deplete similar percentages of cells, at the lower iron values, the hot coated ferrofluid removes significantly more cells.

**Table XII**

| | **Assay for Performance with CEM cells (% removal)** | |
|---|---|---|
| [Fe] ug/ml (final) | FF lot 188-191-15 | FF lot 0395-1308 |
| 0.00 | 0.0 | 0.0 |
| 0.78 | 4.0 | 6.3 |
| 1.56 | 18.3 | 17.1 |
| 3.13 | 71.2 | 47.3 |
| 6.25 | 96.9 | 90.1 |
| 12.5 | 98.7 | 98.1 |

### EXAMPLE 9

### Depletion of Low Density Fraction Mononuclear Cells with Hot Coated Ferrofluid

Mononuclear cells were isolated from a sample of peripheral blood by density centrifugation with Ficoll-Paque ET (Pharmacia Biotech, Uppsala, Sweden), washed twice in 1% BSA/IPBS and suspended in the same buffer to a concentration of approximately 26.5 x 10⁶ cells/ml. Two samples of 0.85 ml each of cell suspension were incubated for 10 minutes at room temperature with 1 ug biotinylated anti-CD45, prepared as in example 18 above. Solutions of streptavidin ferrofluid prepared as in example 6 (lot 188-143-6) were then added to the cells in a volume of 0.85 ml. 10.0 ug and 75 ug of iron/test were used in this experiment. The mixtures were allowed to incubate for 5 minutes at room temperature. Then each sample was pipetted into a 2 ml cell separation chamber as used in example 7 above (Immunicon Corp., Huntingdon Valley, PA). The samples were allowed to magnetically collect as in example 18 for two 7 minute magnetic collections with a pipet resuspension between collections. Efficacy of depletion was determined to be 97.2% with 10.0 ug iron and 97.9% with 75.0 ug of iron.

### EXAMPLE 10

### Coupling Protein A to BSA Ferrofluid

Other proteins can be coupled to the BSA ferrofluids described above. One example is Protein A. BSA ferrofluid was prepared as in example 3 above, except that the total sonication time was 30 minutes. The hot coating was with 600 mg of BSA in 200 ml. After hot coating, the sample was cooled and sonicated for an additional 5 minutes, while cooled in a -4°C ethylene glycol bath. After an overnight incubation of the ferrofluid at 4°C, the ferrofluid was decanted, fractionated with the high field magnet and washed 4 times with 20 mM HEPES, pH 7.5.

BSA ferrofluid was prepared and activated with SMCC as described in Example 6 above. Protein A (Pharmacia Biotech, Uppsala, Sweden) was activated with a Traut's reagent as described in Example 6. Then 1.0 mg ferrofluid and 1.0 mg Protein A were mixed and incubated one hour at room temperature, then overnight at 4°C. The reaction was quenched with mercaptosuccinic acid as described in Example 6, and the ferrofluid was washed 4 times. Binding capacity of this hot coated Protein A ferrofluid was two times higher than the binding capacity of non-heat treated ferrofluid, otherwise prepared identically, and the material was readily filter sterilizable.

### EXAMPLE 11

### Coupling Goat Anti-Mouse to BSA Ferrofluid

Goat anti-mouse antibody can also be coupled to BSA ferrofluid. BSA ferrofluid was prepared and activated with SMCC as described in example 10 above. Goat anti-mouse antibody (Jackson Labs, West Grove, PA) was activated with a Traut's reagent as described in example 10. Then 30.5 mg ferrofluid and 15.6 mg goat anti-mouse antibody were mixed and incubated one hour at room temperature, then overnight at 4°C. The reaction was quenched with mercaptosuccinic acid as described in Example 10, and the ferrofluid was washed 4 times. Ferrofluid prepared in this manner depleted cells more effectively at low ferrofluid concentrations compared to similarly prepared non-heat treated ferrofluids.

### EXAMPLE 12

### Preparation of Hot Coated Ferrofluids Using Polymers Other than BSA

It is also possible to prepare heat-treated ferrofluids with polymers and proteins other than BSA. Polymers such as Dextrans T-10 and T-40 (Pharmacia Biotech, Uppsala, Sweden), and proteins such as beta-lactoglobulin (Sigma, St. Louis, MO) were used in the process described in example 4, except that the hot coating with the BSA was replaced by a hot coating with the above polymers. Ferrofluids prepared by this hot coating process were compared to ferrofluid prepared by an identical process, but without a heating step and compared by size and total adsorbed carbon. In all cases, the hot coated process resulted in small ferrofluids with a relatively high degree of coating, while the cold process resulted in large, unstable particles, which rapidly settled from solution and had significantly less adsorbed coating material.

## Claims

1. A process for making resuspendable, coated magnetic particles by direct application of an aqueous coating material to a particulate magnetic substrate, said process comprising:
a. dividing at least one particle of a magnetic starting material into a plurality of aggregable, smaller sized particles, thereby to provide an uncoated particulate magnetic substrate;
b. forming a suspension of said uncoated particulate magnetic substrate in an aqueous medium; and
c. contacting said uncoated, suspended particulate magnetic substrate with said aqueous coating material, before substantial particulate magnetic substrate aggregation occurs, for.a time sufficient for said coating material to adhere to said substrate, thereby forming said resuspendable, coated magnetic particles.

2. A process according to claim 1 which further comprises heating said particulate magnetic substrate during said contacting step to a temperature of at least about 45°C for a time sufficient for said coating material to adhere to said substrate.

3. A process according to claim 1 or 2, wherein said magnetic starting material comprises at least one transition metal oxide.

4. A process according to claim 1, wherein said magnetic starting material is selected from the group consisting of ZnOFe₂O₃, GdOFe₂O₃, VOFe₂O₃, FeOFe₂O₃, InOFe₂O₃, CuOFe₂O₃, CoOFe₂O₃, MgOFe₂O₃, and Gd₃Fe₅O₁₂.

5. A process according to claim 4, wherein said transition metal oxide is magnetite.

6. A process according to claim 1 or 2, wherein said coating material is a natural or synthetic polymer.

7. A process according to claim 1 or 2, wherein said magnetic starting material is divided under the influence of irradiation, vibration, pH modification, sonication, or a combination thereof.

8. A process according to claim 1 or 2, wherein said coated magnetic particles generally have a maximum dimension of less than 0.2µ.

9. A process according to claim 1 or 2, wherein the weight ratio of said particulate magnetic substrate to said coating material is from about 1000:1 to about 1:10.

10. A process according to claim 1 or 2, further comprising resuspending the magnetic coated particles in water or an aqueous buffer solution.

11. A process according to claim 1 or 2, further including the step of reacting said coated magnetic particles with a bifunctional compound specific to said coating.

12. A process according to claim 1 or 2, further including the step of reacting said coated magnetic particles with a biofunctional compound and reacting the product particles with a bifunctional ligand.

13. A process according to claim 1 or 2, further including the step of reacting said coated magnetic particles with an activating agent and reacting the product particles with a biofunctional ligand.

14. The process according to claim 2, wherein said heating is carried out at a temperature from about 60°C to about 85°C.

15. The process according to claim 1, wherein the magnetic starting material comprises a pair of ions selected from the group consisting of
Co(II)+Ga(III)
Ga(III)+Er(III)
Co(II)+Ru(III)
Ga(III)+Ru(III)
Co(II)+Mn(II)
Ga(III)+Mn(II)
Ga(III)+V(III)
Co(II)+V(III)
Ga(III)+Mo(V)
Ga(III)+Fe(III)
V(III)+Fe(III)
Mn(II)+Ru(III)
V(III)+Mn(II)
Co(II)+Mo(V)
Cr(III)+Ga(III)
Cr(III)+Mn(II)
Er(III)+Ru(III)
Er(III)+Co(II)
Mn(II)+Er(III)
Cr(III)+Fe(II).

16. A process according to claim 1 wherein step (a) comprises sonication of said magnetic starting material at a temperature of about 75°C, for a time sufficient to produce particles in the size range of 40-180 nm;
and step (c) is performed at a temperature in the range of 70°-80°C.

17. A process according to claim 16, wherein said coating material is selected from the group consisting of protein, peptide and nucleic acid.

18. A process according to claim 16 or claim 17, wherein said uncoated, suspended particulate magnetic substrate is contacted with said coating material in an amount no greater that that which is sufficient to provide complete coverage of said resuspendable, coated magnetic particles.

19. Resuspendable coated magnetic particles prepared by the process of claims 16 or claim 17.

20. A resuspendable coated magnetic particle exhibiting colloidal stability in a 1 molar salt solution, as indicated by an increase in particle size of no greater than two-fold upon exposure of said particle to said salt solution for a period of up to 24 hours at room temperature.

## Patentansprüche

1. Verfahren zur Herstellung von resuspendierbaren, beschichteten Magnetteilchen durch direktes Auftragen eines wässrigen Beschichtungsmaterials auf ein partikuläres magnetisches Substrat, wobei das Verfahren Folgendes umfasst:
a) Teilen zumindest eines Teilchens eines magnetisches Ausgangsmaterials in mehrere aggregierbare, kleinere Teilchen, um so ein unbeschichtetes, partikuläres magnetisches Substrat bereitzustellen;
b) Bilden einer Suspension dieses unbeschichteten, partikulären magnetischen Substrats in einem wässrigen Medium; und
c) Kontaktieren des unbeschichteten, partikulären magnetischen Substrats mit dem wässrigen Beschichtungsmaterial, bevor das partikuläre magnetische Substrat nennenswerte Aggregation erfährt, und zwar für einen Zeitraum, der ausreicht, damit das Beschichtungsmaterial am Substrat haftet, wodurch die resuspendierbaren, beschichteten Magnetteilchen gebildet werden.

2. Verfahren nach Anspruch 1, das außerdem das Erhitzen des partikulären magnetischen Substrats während des Kontaktierungsschritts auf eine Temperatur von zumindest 45 °C umfasst, und zwar für einen Zeitraum, der ausreicht, damit das Beschichtungsmaterial am Substrat haftet.

3. Verfahren nach Anspruch 1 oder 2, worin das magnetische Ausgangsmaterial zumindest ein Übergangsmetalloxid umfasst.

4. Verfahren nach Anspruch 1, worin das magnetische Ausgangsmaterial aus der aus ZnOFe₂O₃, GdOFe₂O₃, VOFe₂O₃, FeOFe₂O₃, InOFe₂O₃, CuOFe₂O₃, CoOFe₂O₃, MgOFe₂O₃ und Gd₃Fe₅O₁₂ bestehenden Gruppe ausgewählt ist.

5. Verfahren nach Anspruch 4, worin das Übergangsmetalloxid Magnetit ist.

6. Verfahren nach Anspruch 1 oder 2, worin das Beschichtungsmaterial ein natürliches oder synthetisches Polymer ist.

7. Verfahren nach Anspruch 1 oder 2, worin das magnetische Ausgangsmaterial unter Einfluss von Strahlung, Schwingungen, pH-Modifikation, Beschallung oder einer Kombination davon geteilt wird.

8. Verfahren nach Anspruch 1 oder 2, worin die beschichteten Magnetteilchen im Allgemeinen eine maximale Größe von weniger als 0,2 µm aufweisen.

9. Verfahren nach Anspruch 1 oder 2, worin das Gewichtsverhältnis zwischen dem partikulären magnetischen Substrat und dem Beschichtungsmaterial etwa 1.000:1 bis etwa 1:10 beträgt.

10. Verfahren nach Anspruch 1 oder 2, das außerdem das Resuspendieren der beschichteten Magnetteilchen in Wasser oder einer wässrigen Pufferlösung umfasst.

11. Verfahren nach Anspruch 1 oder 2, das außerdem den Schritt des Umsetzens der beschichteten Magnetteilchen mit einer für die Beschichtung spezifischen bifunktionellen Verbindung umfasst.

12. Verfahren nach Anspruch 1 oder 2, das außerdem den Schritt des Umsetzens der beschichteten Magnetteilchen mit einer bifunktionellen Verbindung und des Umsetzens der Produktteilchen mit einem bifunktionellen Liganden umfasst.

13. Verfahren nach Anspruch 1 oder 2, das außerdem den Schritt des Umsetzens der beschichteten Magnetteilchen mit einem Aktivator und des Umsetzens der Produktteilchen mit einem bifunktionellen Liganden umfasst.

14. Verfahren nach Anspruch 2, worin das Erhitzen bei einer Temperatur von etwa 60 °C bis etwa 85 °C durchgeführt wird.

15. Verfahren nach Anspruch 1, worin das magnetische Ausgangsmaterial ein lonenpaar umfasst, das aus der folgenden Gruppe ausgewählt ist:
Co(II)+Ga(III)
Ga(III)+Er(III)
Co(II)+Ru(III)
Ga(III)+Ru(III)
Co(II)+Mn(II)
Ga(III)+Mn(II)
Ga(III)+V(III)
Co(II)+V(III)
Ga(III)+Mo(V)
Ga(III)+Fe(III)
V(III)+Fe(III)
Mn(II)+Ru(III)
V(III)+Mn(II)
Co(II)+Mo(V)
Cr(III)+Ga(III)
Cr(III)+Mn(II)
Er(III)+Ru(III)
Er(III)+Co(II)
Mn(II)+Er(III)
Cr(III)+Fe(II).

16. Verfahren nach Anspruch 1, worin Schritt (a) eine Beschallung des magnetischen Ausgangsmaterials bei einer Temperatur von etwa 75 °C umfasst, und zwar ausreichend lange, um Teilchen in einem Größenbereich von 40 bis 180 nm herzustellen;
und Schritt (c) bei einer Temperatur im Bereich von 70 bis 80 °C durchgeführt wird.

17. Verfahren nach Anspruch 16, worin das Beschichtungsmaterial aus der aus Protein, Peptid und Nucleinsäure bestehenden Gruppe ausgewählt ist.

18. Verfahren nach Anspruch 16 oder 17, worin das unbeschichtete, suspendierte, partikuläre magnetische Substrat mit einer Menge des Beschichtungsmaterials kontaktiert wird, die nicht größer ist als jene, die ausreicht, um die resuspendierbaren, beschichteten Magnetteilchen vollkommen zu bedecken.

19. Resuspendierbare, beschichtete Magnetteilchen, die nach einem Verfahren nach Anspruch 16 oder 17 hergestellt wurden.

20. Resuspendierbares, beschichtetes Magnetteilchen, das in einer einmolaren Salzlösung kolloidale Stabilität aufweist, was sich anhand einer Zunahme der Teilchengröße um nicht mehr als das Zweifache zeigt, wenn das Teilchen für einen Zeitraum von bis zu 24 h bei Raumtemperatur dieser Salzlösung ausgesetzt wird.

## Revendications

1. Procédé de fabrication de particules magnétiques enrobées, pouvant être remises en suspension, par application directe d'un matériau aqueux de revêtement à un substrat magnétique particulaire, ledit procédé comprenant:
a. la division d'au moins une particule d'une matière première magnétique en un certain nombre de particules plus petites agrégeables pour ainsi produire un substrat magnétique particulaire non enrobé;
b. la formation d'une suspension dudit substrat magnétique particulaire non enrobé dans un milieu aqueux; et
c. la mise en contact dudit substrat magnétique particulaire non enrobé en suspension avec ledit matériaux aqueux de revêtement avant que l'agrégation sensible du substrat magnétique particulaire ne se produise, pendant un temps suffisant pour que ledit matériau de revêtement adhère audit substrat pour ainsi former lesdites particules magnétiques enrobées pouvant être remises en suspension.

2. Procédé selon la revendication 1 qui comprend de plus le chauffage dudit substrat magnétique particulaire pendant ladite étape de mise en contact à une température d'au moins environ 45°C pendant un temps suffisant pour que ledit matériau de revêtement adhère audit substrat.

3. Procédé selon la revendication 1 ou 2, où ladite matière première magnétique comprend au moins un oxyde d'un métal de transition.

4. Procédé selon la revendication 1, où ladite matière première magnétique est sélectionnée dans le groupe consistant en ZnOFe₂O₃, GdOFe₂O₃, VOFe₂O₃, FeOFe₂O₃, InOFe₂O₃, CuOFe₂O₃, CoOFe₂O₃, MgOFe₂O₃, et Gd₃Fe₅O₁₂.

5. Procédé selon la revendication 4, où ledit oxyde d'un métal de transition est de la magnétite.

6. Procédé selon la revendication 1 ou 2, où ledit matériau de revêtement est un polymère naturel ou synthétique.

7. Procédé selon la revendication 1 ou 2, où ladite matière première magnétique est divisée sous l'influence d'un rayonnement, de vibrations, d'une modification du pH, d'une sonication, ou d'une combinaison de cela.

8. Procédé selon la revendication 1 ou 2, où lesdites particules magnétiques enrobées ont généralement une dimension maximale de moins de 0,2 µ.

9. Procédé selon la revendication 1 ou 2, où le rapport en poids dudit substrat magnétique particulaire audit matériau de revêtement est d'environ 1000:1 à environ 1:10.

10. Procédé selon la revendication 1 ou 2, comprenant de plus la remise en suspension des particules magnétiques enrobées dans l'eau ou une solution aqueuse d'un tampon.

11. Procédé selon la revendication 1 ou 2, comprenant de plus l'étape de faire réagir lesdites particules magnétiques enrobées avec un composé bifonctionnel spécifique dudit revêtement.

12. Procédé selon la revendication 1 ou 2, comprenant de plus l'étape de faire réagir lesdites particules magnétiques enrobées avec un composé biofonctionnel et de faire réagir les particules produites avec un ligand bifonctionnel.

13. Procédé selon la revendication 1 ou 2, comprenant de plus l'étape de faire réagir lesdites particules magnétiques enrobées avec un agent activant et de faire réagir les particules produites avec un ligand biofonctionnel.

14. Procédé selon la revendication 2, où ledit chauffage est effectué à une température d'environ 60°C à environ 85°C.

15. Procédé selon la revendication 1, où la matière première magnétique comprend une paire d'ions sélectionnée dans le groupe consistant en
Co(II)+Ga(III)
Ga(III)+Er(III)
Co(II)+Ru(III)
Ga(III)+Ru(III)
Co (II)+Mn(II)
Ga(III)+Mn(II)
Ga(III)+V(III)
Co(II)+V(III)
Ga(III)+Mo(V)
Ga(III)+Fe(III)
V(III)+Fe(III)
Mn(II)+Ru(III)
V(III)+Mn(II)
Co(II)+Mo(V)
Cr(III)+Ga(III)
Cr(III)+Mn(II)
Er(III)+Ru(III)
Er(III)+Co(II)
Mn(II)+Er(III)
Cr(III)+Fe(II).

16. Procédé selon la revendication 1, où l'étape (a) comprend la sonication de ladite matière première magnétique à une température d'environ 75°C, pendant un temps suffisant pour produire des particules dans une gamme de dimensions de 40-180 nm;
et l'étape (c) est accomplie à une température dans la gamme de 70-80°C.

17. Procédé selon la revendication 16, où ledit matériau d'enrobage est sélectionné dans le groupe consistant en protéine, peptide et acide nucléique.

18. Procédé selon la revendication 16 ou la revendication 17, où ledit substrat magnétique particulaire non enrobé, en suspension est mis en contact avec ledit matériau de revêtement en une quantité qui ne dépasse pas celle qui est suffisante pour produire une couverture complète desdites particules magnétiques enrobées pouvant être remises en suspension.

19. Particules magnétiques enrobées pouvant être remises en suspension préparées par le procédé des revendications 16 ou de la revendication 17.

20. Particule magnétique enrobée pouvant être remise en suspension présentant une stabilité colloïdale dans une solution de sel à 1 molaire, comme indiqué par une augmentation de la dimension des particules de pas plus de deux fois lors d'une exposition de ladite particule à ladite solution de sel pendant une période pouvant atteindre 24 heures à température ambiante.
